# EUROPEAN PATENT APPLICATION

(11) **EP 4 285 905 A1**
(43) Date of publication of application: **06.12.2023**
(21) Application number: 22382541.5
(22) Date of filing: 03.06.2022
(51) Int. Cl.: A61K 31/48, A61P 35/00

(54) **CABERGOLINE OR A PHARMACEUTICALLY ACCEPTABLE SALT OR SOLVATE THEREOF, FOR USE IN PREVENTION OR DELAY OF POST-PREGNANCY BREAST CANCER IN A FEMALE MAMMAL**

(71) Applicant: Consejo Superior de Investigaciones Cientificas, 28006 Madrid (ES)
(72) Inventor: PÉREZ LOSADA, Jesús, Madrid (ES); BLANCO GÓMEZ, Adrián, Madrid (ES); GARCÍA SANCHA, Natalia, Madrid (ES); CORCHADO COBOS, Roberto, Madrid (ES)
(74) Representative: Manuel Illescas y Asociados, S.L.

(57) **Abstract**

The present invention relates to cabergoline or a pharmaceutically acceptable salt or solvate thereof, for use in prevention or delay of post-pregnancy breast cancer in a female mammal. Analogously, the present invention relates to a method of prevention or delay of post-pregnancy breast cancer in a female mammal comprising administration thereto of cabergoline or a pharmaceutically acceptable salt or solvate thereof. Similarly, the present invention relates to use of cabergoline or a pharmaceutically acceptable salt or solvate thereof for the manufacture of a medicament for prevention or delay of post-pregnancy breast cancer in a female mammal. The present invention also relates to cabergoline or a pharmaceutically acceptable salt or solvate thereof, for use in prevention or delay of appearance or development of post-pregnancy breast cancer in a female mammal, wherein said female mammal has weaned their offspring and/or is undergoing postlactational involution.

## Description

### Field of the invention

The present invention relates to the field of oncology, in particular to the field of cancer prevention or delay.

### Background

Chemoprevention strategies for breast cancer that have fewer side-effects or where side-effects are not as serious, and/or wherein the duration of administration is short (ideally single dose) so that there would be no problems with adherence to administration, as well as wherein the effects of chemoprevention are permanent and avoid the need for amputation or other surgical techniques are desired.

It is the problem solved by the present invention to prevent or delay post-pregnancy breast cancer in a female mammal. It is also the problem solved by the present invention to prevent or delay post-pregnancy breast cancer in a female mammal who has weaned their offspring and/or is undergoing postlactational involution and/or has a mutation in the *Brca1* gene and/or the *P53* gene. It is furthermore the problem solved by the present invention to prevent or delay post-pregnancy breast cancer in a woman, preferably a woman who is ≥ 30 years old when having first given birth more preferably ≥ 35 years old when having first given birth, and/or a woman who was pregnant for more than 34 weeks. Thus, it is also the problem solved by the present invention to prevent or delay post-pregnancy breast cancer in a female mammal with least 34 weeks of gestation (34 weeks post-gestation).

### Brief description of the invention

The present invention relates to cabergoline or a pharmaceutically acceptable salt or solvate thereof, for use in prevention or delay of post-pregnancy breast cancer in a female mammal.

Analogously, the present invention relates to a method of prevention or delay of post-pregnancy breast cancer in a female mammal comprising administration thereto of cabergoline or a pharmaceutically acceptable salt or solvate thereof. Similarly, the present invention relates to use of cabergoline or a pharmaceutically acceptable salt or solvate thereof for the manufacture of a medicament for prevention or delay of post-pregnancy breast cancer in a female mammal.

The present invention also relates to cabergoline or a pharmaceutically acceptable salt or solvate thereof, for use in prevention or delay of appearance or development of post-pregnancy breast cancer in a female mammal, wherein said female mammal has weaned their offspring and/or is undergoing postlactational involution.

Analogously, the present invention relates to a method of prevention or delay of appearance or development of post-pregnancy breast cancer in a female mammal comprising administration thereto of cabergoline or a pharmaceutically acceptable salt or solvate thereof, wherein said female mammal has weaned their offspring and/or is undergoing postlactational involution. Similarly, the present invention relates to use of cabergoline or a pharmaceutically acceptable salt or solvate thereof, for the manufacture of a medicament for prevention or delay of appearance or development of post-pregnancy breast cancer in a female mammal, wherein said female mammal has weaned their offspring and/or is undergoing postlactational involution.

### Description of the figures

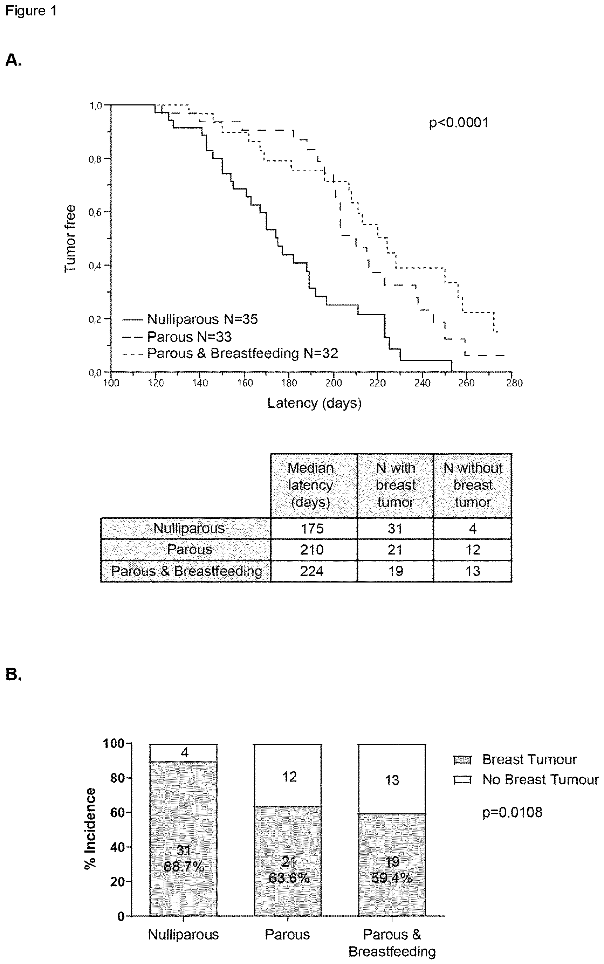

**Figure 1****.** Pregnancy increases resistance to breast cancer in the *Brca1*/*P53* deficient model. Kaplan-Meier curves and the log-rank test, comparing increased tumour latency in Brca1/P53-deficient multiparous mice with nulliparous mice, wherein **A.** shows comparison of nulliparous mice vs. non-lactating (= non-breastfeeding, meaning herein that lactation never occurred) multiparous mice vs. lactating (= breastfeeding) multiparous mice. The protective effect was observed in both lactating and non-lactating mice (two pregnancies in both groups); and **B.** Chi-square test results showing a lower tumour incidence was observed in both groups of multiparous mice with respect to nulliparous mice. No differences were observed between lactating and non-lactating mice.

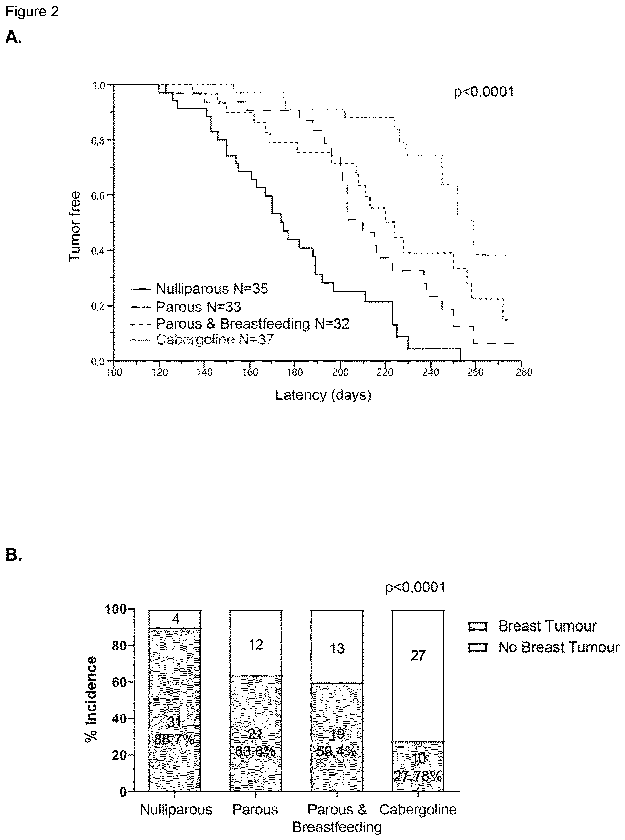

**Figure 2****.** Cabergoline potentiates the protective effect of pregnancy in the *Brca1*/*P53* deficient mouse model. **A.** Kaplan-Meier curves and log-rank test showing increased tumour latency in multiparous mice deficient in *Brca1*/*P53* treated with cabergoline when compared with respect to lactating (breastfeeding) multiparous mice, non-lactating (non-breastfeeding) multiparous mice and nulliparous mice; and **B.** Chi-square test results showing comparison of tumour incidence in multiparous mice deficient in *Brca1*/*P53* treated with cabergoline, lactating multiparous mice, non-lactating multiparous mice and nulliparous mice.

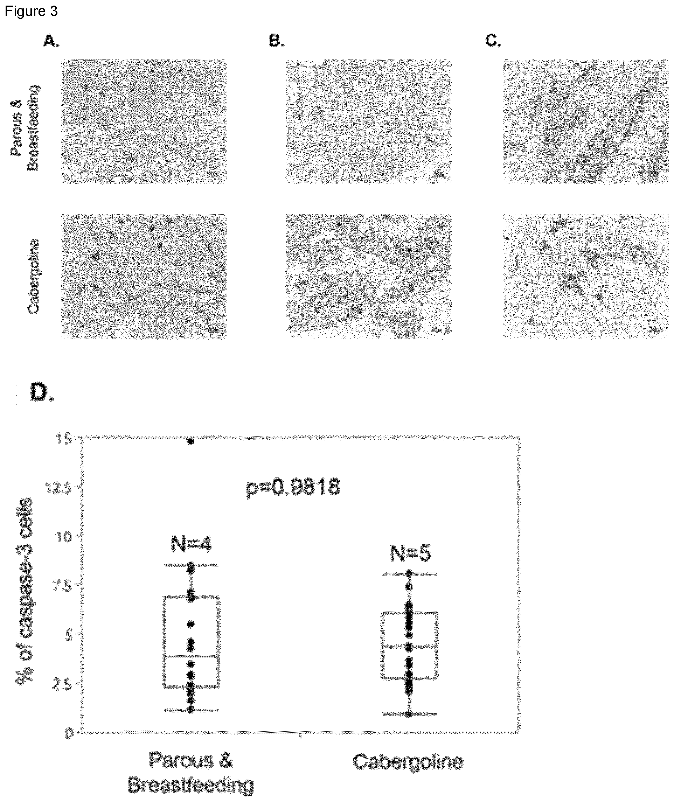

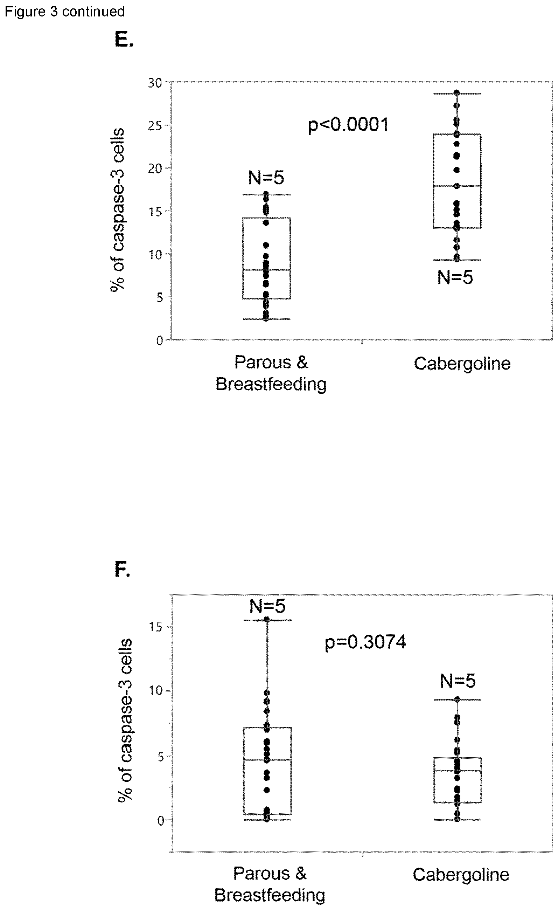

**Figure 3****.** Cabergoline potentiates post-lactational involution in Brca1/P53-deficient mice. Images of apoptotic cells, positive against caspase 3, visualized with immunohistochemistry at **A.** 1 day of post-lactational involution; **B.** 3 days of post-lactational involution; and **C.** 7 days of post-lactational involution, wherein upper panels show samples obtained from lactating (breastfeeding) multiparous mice and lower panels show samples obtained from lactating multiparous mice that were additionally treated with cabergoline; Mann-Whitney U test results on the percentage of apoptotic cells positive against caspase 3, detected and quantified following said visualization in lactating multiparous mice (Parous and Breastfeeding) and lactating multiparous mice treated with cabergoline (Cabergoline), at **D.** 1 day of post-lactational involution; **E.** 3 days of post-lactational involution; and **F.** 7 days of post-lactational involution, wherein quantification was performed with the Leica Application Suite software.

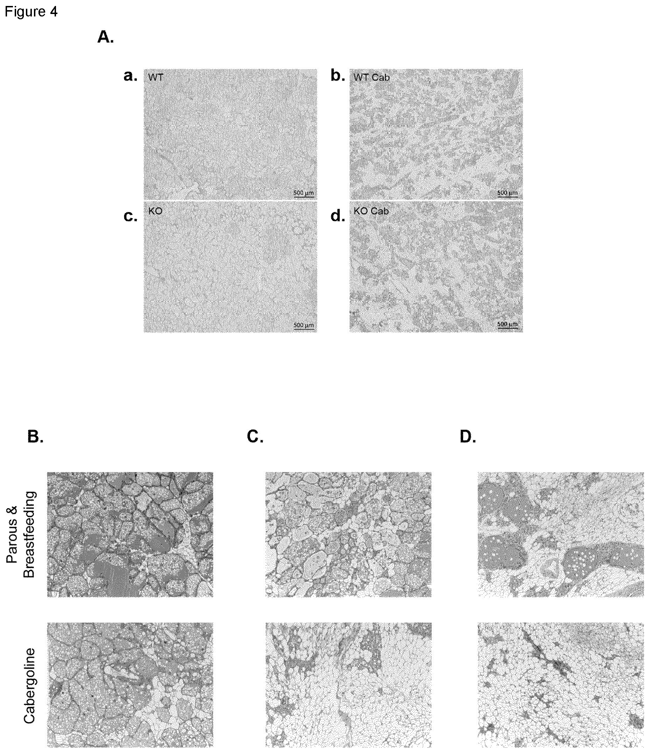

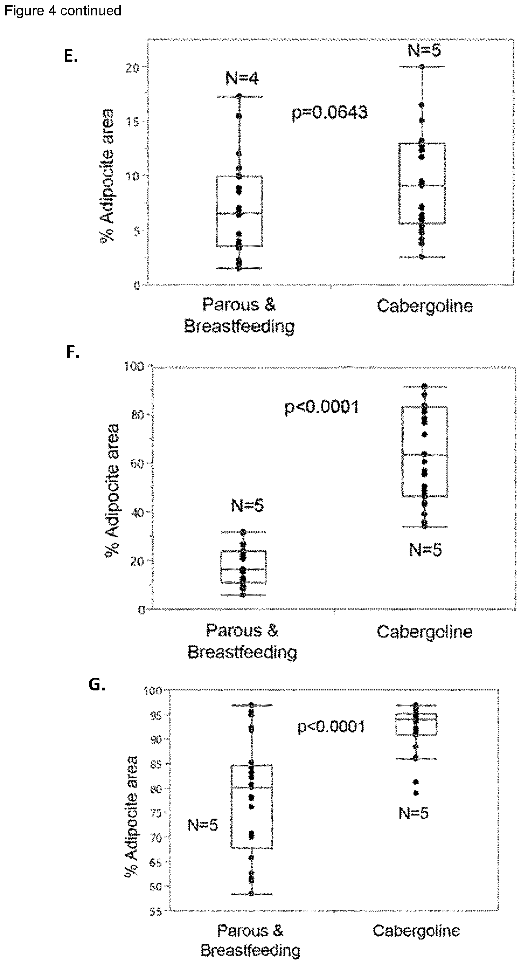

**Figure 4****.** Cabergoline accelerates histological changes characteristic of post-lactational involution in *Brca1*/*P53*-deficient mice (N = 5 mice per group). **A.** Images of detail of samples obtained at 3 days of post-lactational involution in wild-type mice (WT, upper panels) and mice deficient in *Brca1*/*P53* (KO, lower panels) without treatment (panels a and c, as controls) and following administration of cabergoline (panels b and d). Note that the glands of mice not treated with cabergoline are full of ducts; while, in the treated mice, adipose tissue is observed between the ducts; hematoxylin-eosin staining to assess the area of adipose tissue at **B.** 1 day of post-lactational involution; **C.** 3 days of post-lactational involution; **D.** 7 days of post-lactational involution, wherein upper panels show samples obtained from lactating (breastfeeding) multiparous mice and lower panels show samples obtained from lactating multiparous mice that were additionally treated with cabergoline; Mann-Whitney U test results on the percentage area of adipocytes in lactating multiparous mice (Parous and Breastfeeding) and lactating multiparous mice treated with cabergoline (Cabergoline), detected and quantified following said visualization, at **E.** 1 day of post-lactational involution; **F.** 3 days of post-lactational involution; and **G.** 7 days of post-lactational involution, wherein quantification was performed with ImageJ software.

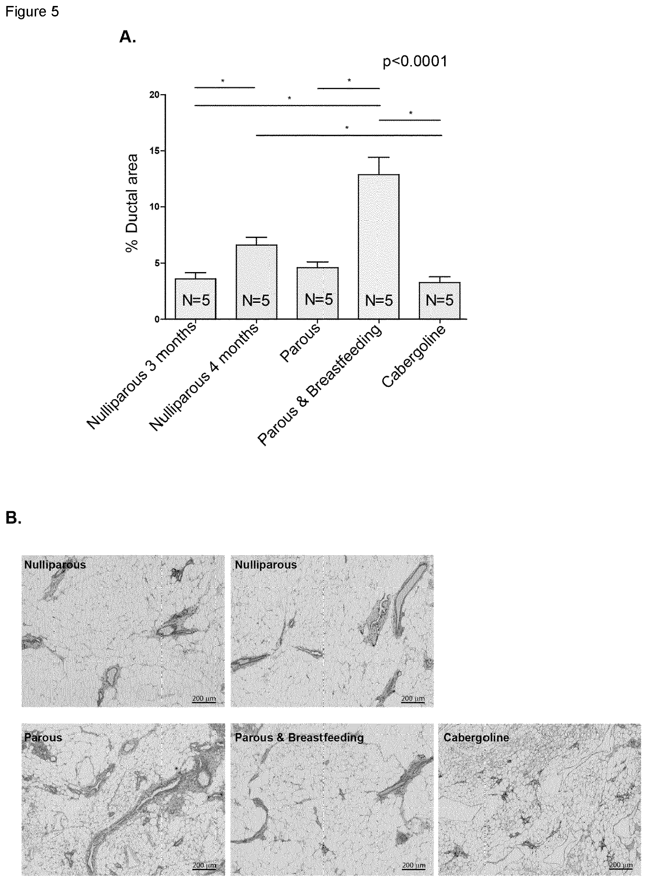

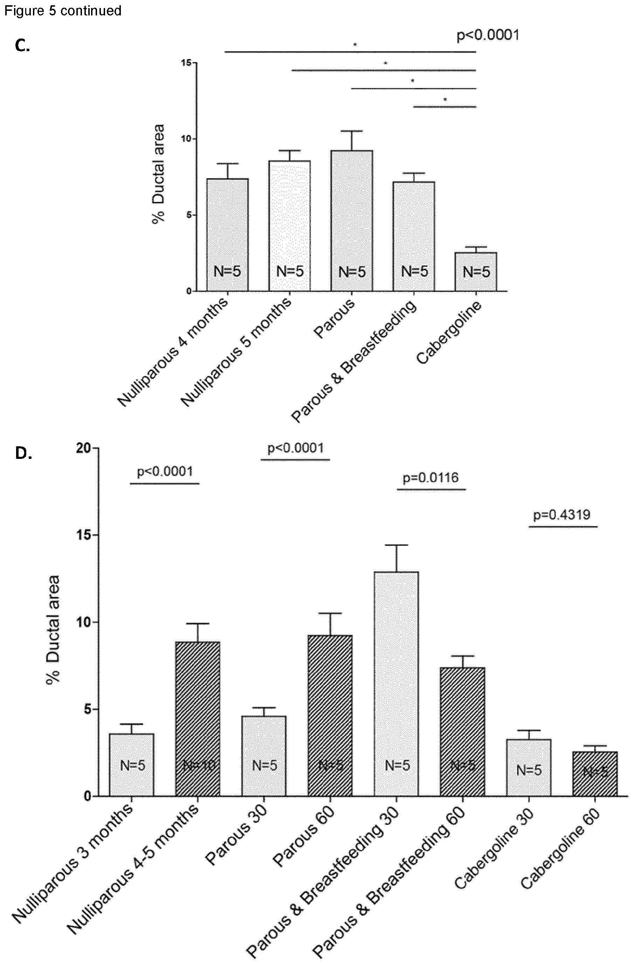

**Figure 5****.** Cabergoline produces a long-term reduction in the epithelial component of the mammary gland. **A.** Dunn post-hoc test showing comparison of the percentage ductal area 30 days after post-lactation involution, wherein in nulliparous mice there are two age groups, 3 months (Null 3 months), which are equivalent in age to non-lactating (non-breastfeeding) multiparous mice 30 days after weaning (Parous); and 4 months (Null 4 months), which are equivalent in age to lactating (breastfeeding) multiparous mice at 30 days after weaning (P&B), this latter group also being that which was treated with cabergoline (Cabergoline); **B.** Images obtained following hematoxylin-eosin staining of samples taken 60 days post-weaning from non-tumorous breasts of nulliparous mice (upper left panel, Nulliparous) and non-lactating (non-breastfeeding) multiparous mice (lower left panel, Parous) at 4 months, nulliparous mice (upper central panel, Nulliparous) and lactating (breastfeeding) multiparous mice at 5 months (lower central panel, P&B) and lactating (breastfeeding) multiparous mice treated with cabergoline (lower right panel, Cabergoline); **C.** Dunn post-hoc test showing comparison of the percentage ductal area at 60 days post-weaning in non-tumorous breasts of nulliparous mice (upper left panel) and non-lactating (non-breastfeeding) multiparous mice (lower left panel) at 4 months (Null 4 months), nulliparous mice (upper central panel) and lactating (breastfeeding) multiparous mice at 5 months (lower central panel) and lactating multiparous mice treated with cabergoline (lower right panel), wherein quantification was performed with ImageJ software; **D.** Mann-Whitney U test results on comparison of the ductal area at 30 and 60 days of post-lactational involution, wherein quantification was performed with ImageJ software. As there were no significant differences between nulliparous mice at 4 and 5 months (cf. **C.),** these samples were pooled.

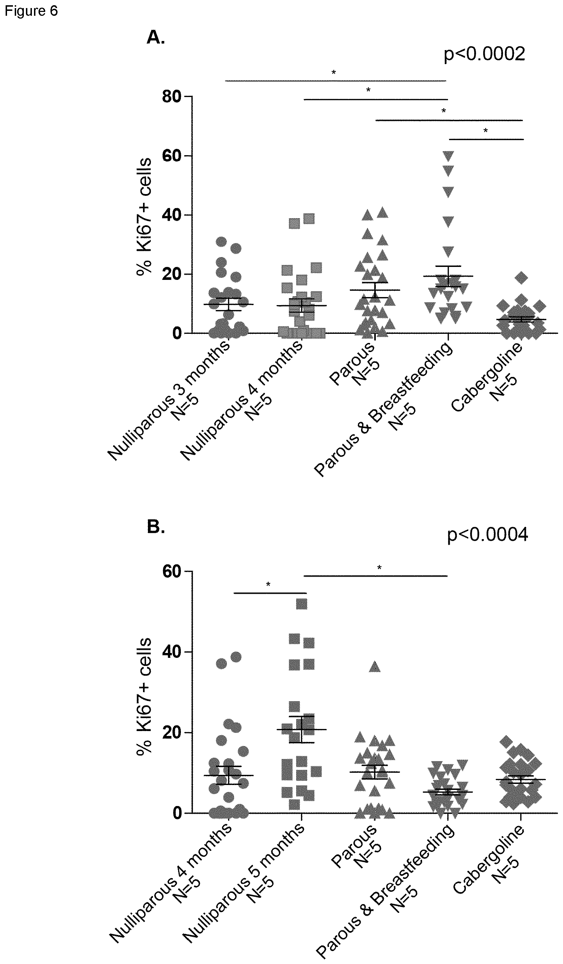

**Figure 6****.** Cabergoline accelerates the appearance of a hypoproliferative state in the mammary epithelium. ANOVA test followed by Dunn's post hoc test results in mice (N = 5 mice per group) for cellular proliferation (as a % of Ki67+ cells, wherein proliferation was detected by immunolabeling against Ki-67 and quantified with the Leica Application Suite LAS V.3.7 software), whereby **A.** mice treated with cabergoline show a lower basal proliferation of the epithelium at 30 days of post-lactational involution than in nulliparous mice at 3 months, nulliparous mice at 4 months, non-lactating (non-breastfeeding) multiparous mice 30 days after weaning (i.e. at 30 days of post-lactational involution) and lactating (breastfeeding) multiparous mice at 30 days after weaning; and **B.** pregnancy induced a hypoproliferative state in mice treated with cabergoline at 60 days of post-lactational involution, in non-lactating (non-breastfeeding) multiparous mice 60 days after weaning (i.e. at 60 days of post-lactational involution) and lactating (breastfeeding) multiparous mice at 60 days after weaning, however, in Brca1/P53-deficient nulliparous mice, a progressive increase in epithelial proliferation is seen.

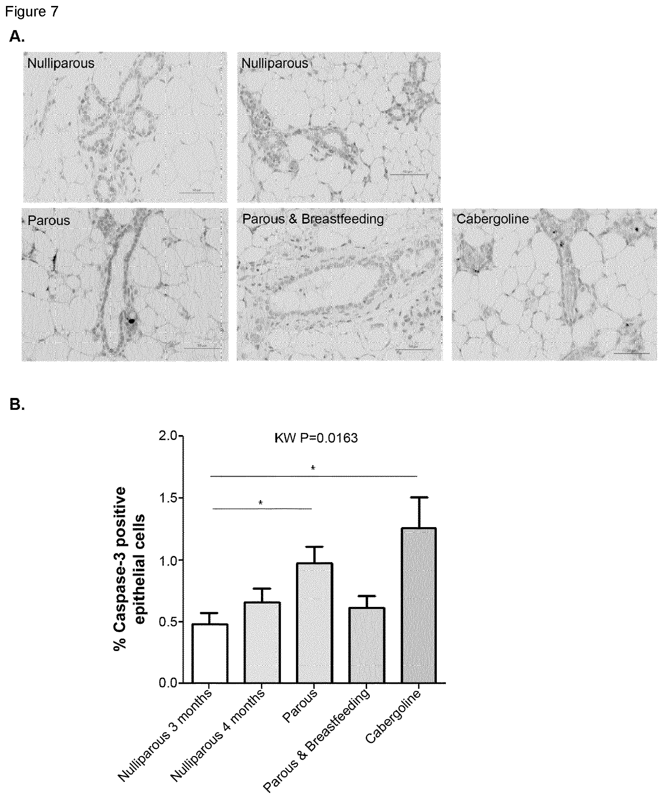

**Figure 7****. A.** Images showing basal apoptosis in the non-tumorous mammary epithelium of untreated nulliparous and multiparous Brca1/P53-deficient mice and multiparous Brca1/P53-deficient mice treated with cabergoline, obtained from samples taken 30 days post-weaning from non-tumorous breasts of nulliparous mice (upper left panel, Nulliparous) and non-lactating (non-breastfeeding) multiparous mice (lower left panel, Parous) at 3 months, nulliparous mice (upper central panel, Nulliparous) and lactating (breastfeeding) multiparous mice at 4 months (lower central panel, P&B) and lactating multiparous mice treated with cabergoline (lower right panel, Cabergoline), whereby the latter show greater basal apoptosis in the epithelial component at 30 days post-weaning, wherein apoptosis was detected by immunolabeling against caspase 3 and quantified with the Leica Application Suite LAS V.3.7 software; and **B.** ANOVA test followed by Dunn post-hoc test results for nulliparous mice at 3 months (3 months Nul), nulliparous mice at 4 months (4 months Nul), non-lactating (non-breastfeeding) multiparous mice 30 days after weaning (i.e. at 30 days of post-lactational involution, Parous) and lactating (breastfeeding) multiparous mice (P&B) at 30 days after weaning, as well as lactating multiparous mice treated with cabergoline at 30 days after weaning (Cabergoline).

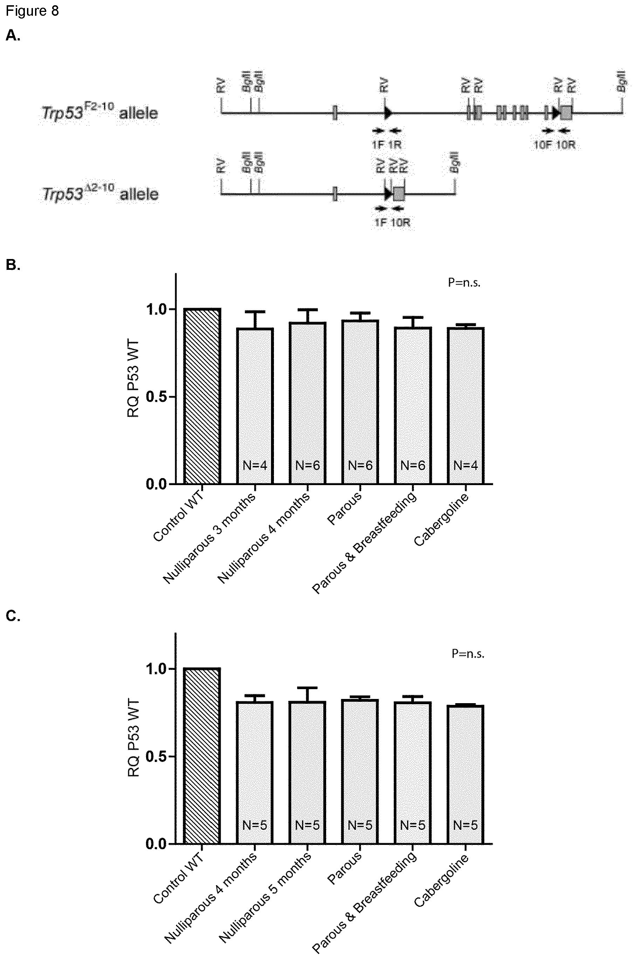

**Figure 8****.** The proportion of recombinant cells is maintained at 30 and 60 days post-weaning (i.e. at 30 and 60 days post-lactational involution), which indirectly indicates a reduction in the absolute number of recombinant cells in the groups with reduced epithelial component. **A.** Diagram of the recombination of the *P53* allele in the mouse model used. Quantification of the WT allele using the Delta-Delta CT method at **B.** 30 days post-lactational involution and **C.** 60 days post-lactational involution, using qPCR and TaqMan probes. No differences were seen between the groups. Control is WT tissue with 100% cells without recombination.

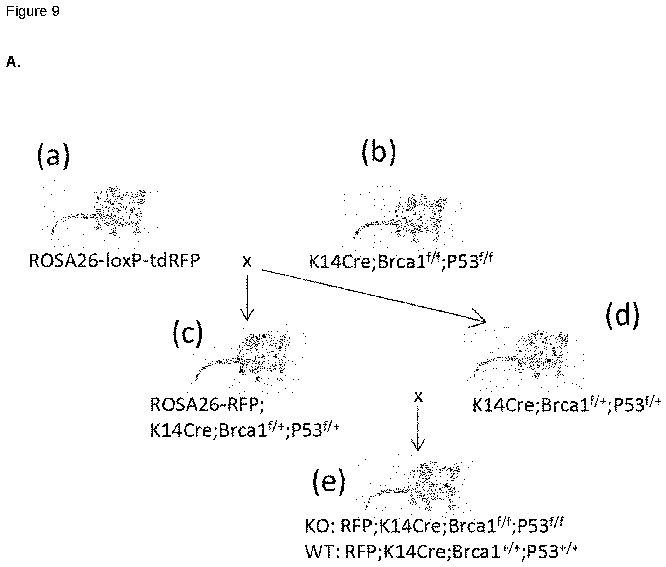

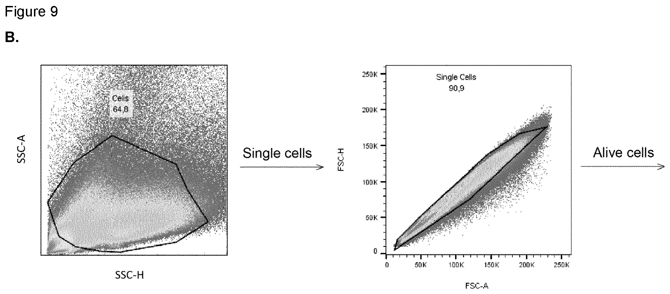

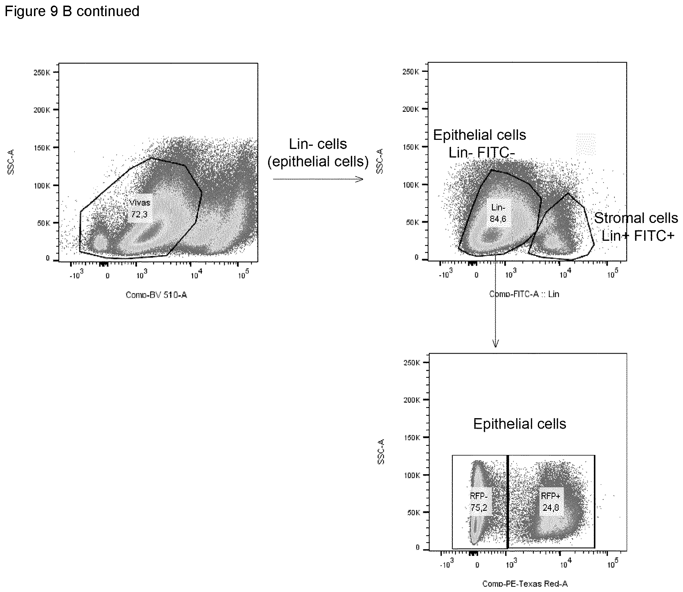

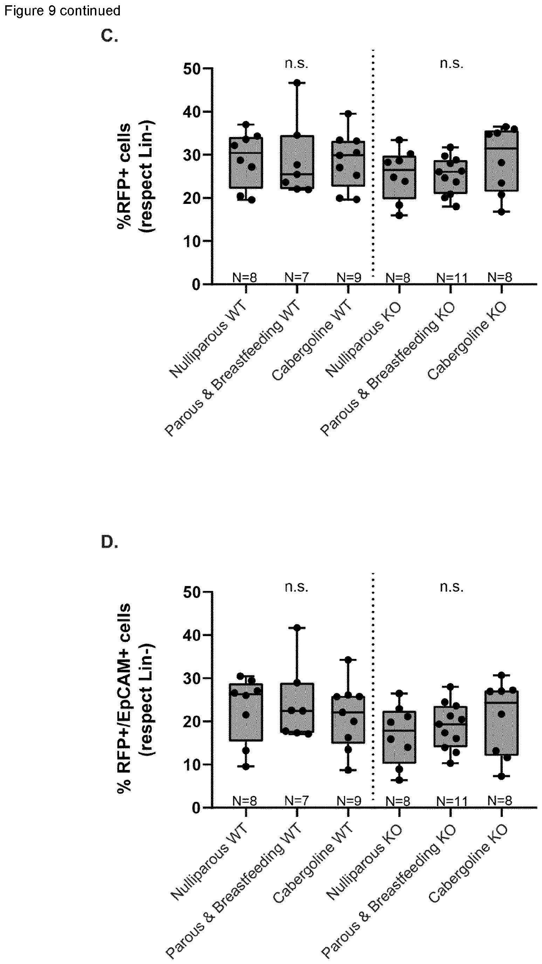

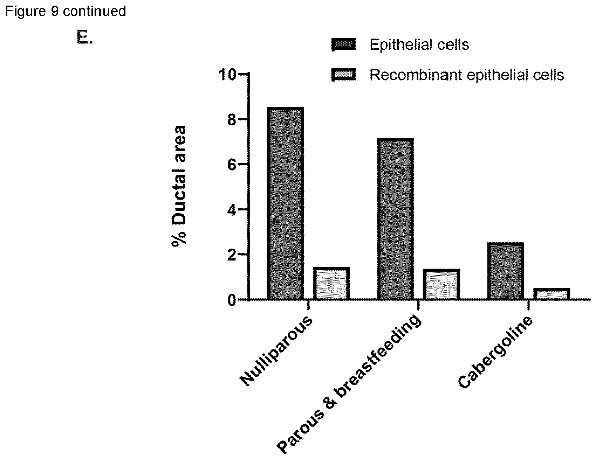

**Figure** 9. The quantification of the recombinant cells with fluorescence at 60 days of involution confirms the lack of statistically significant differences in their proportion between groups. A. Diagram of the crossing of mice to obtain mice in which the recombinant cells emit red fluorescence, wherein (a) the "fluorescent" (RFP) mouse expresses the discosome Red Fluorescent Protein (dsRed) in a tandem dimer (dtRFP) under the ROSA26 promoter and the RFP cDNA is preceded by a stop codon; these mice were crossed with (b) the *Brca1*/*P53* deficient mouse, which is both transgenic for Cre recombinase under the cytokeratin 14 promoter (K14-Cre), which eliminates *Brca1* and *P53* in a percentage of the breast cells; (c) in the F1 generation, transgenic mice for K14-Cre are obtained that remove the stop promoter and cause RFP to be expressed, whereby Cre recombinase simultaneously removes the *Brca1* and *P53* alleles, which remain heterozygous; (d) among the F1 offspring there are also mice heterozygous for the loss of *Brca1* and *P53*; and (e) mice from (c) and (d) are crossed to obtain mice that lose both alleles of *Brca1* and *P53.* RFP+ mice without loss of the Brca1/P53 alleles were also generated; B. Steps for the quantification of RFP+ breast cells by flow cytometry. The tissue was previously crushed and digested, and the adipose component was eliminated by generating organoids. After staining with antibodies, the samples were passed through the cytometer. In analysis (a), events with estimated cell size were chosen, and fragments and clumps were avoided; (b) single cells were picked; (c) live cells were selected (negative for a fluorescent marker of death); (d) FITC (fluorescein isothiocyanate)-conjugated antibody-labeled stromal cells (emits green fluorescence) or lineage (Lin) positive cells (includes fibroblast (CD140+), leukocyte (CD45+) and endothelial cell (CD34+) lineages) were removed; (e) in the cells that remain (the FITC-Lin-) the epithelial cells of the breast were found by exclusion, and RFP+ (recombinant) and RFP- (non-recombinant) epithelial populations were determined; C. Kruskal-Wallis test showing comparison of the ratio of recombinant cells (RFP+) to epithelial cells (Lin-); D. Kruskal-Wallis test showing comparison of the proportion of recombinant cells (EpCAM+RFP+) compared to epithelial cells (Lin-); and E. Estimation of the absolute reduction of the recombinant cells based on the observed reduction of the ductal component in **Figure 5****.**

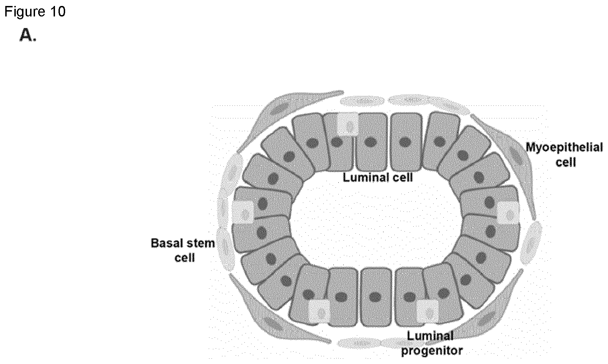

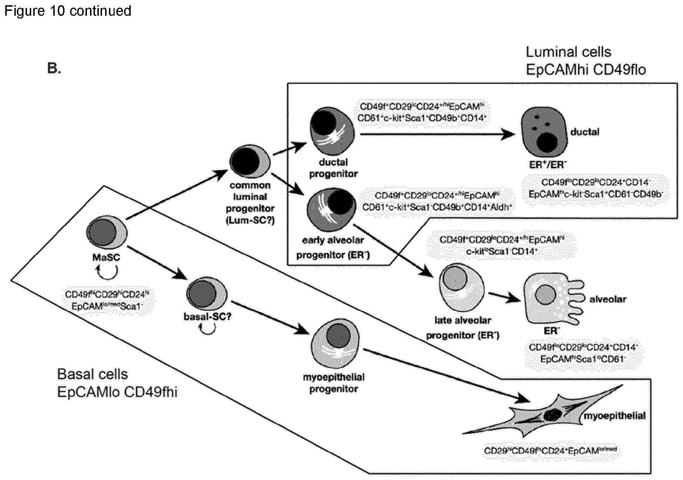

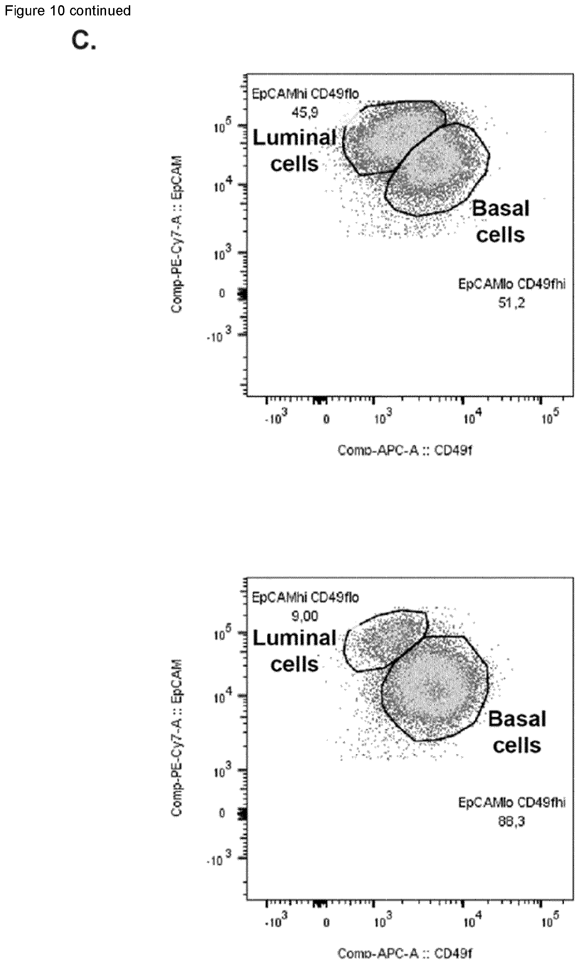

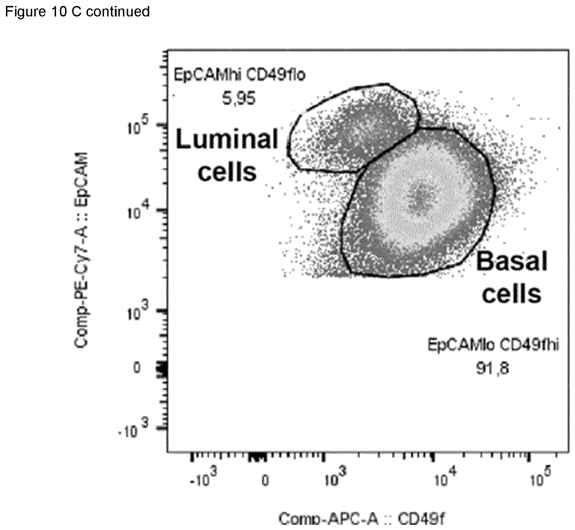

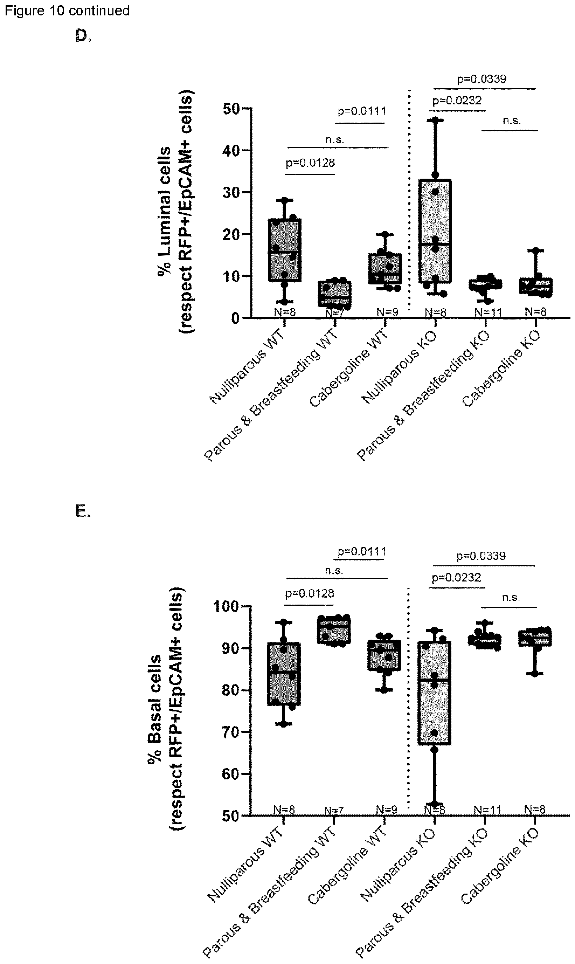

**Figure 10****.** Pregnancy produces a decrease in the proportion of the luminal component within the recombinant cells. A. Diagram of a breast duct, wherein luminal and myoepithelial or basal cells are identified, and the precursors of both. The stem cells of the breast, poorly defined, would be found within the basal component; **B.** Diagram of the cellular differentiation of the mammary gland. The two large cell subpopulations are identified: luminal (upper right frame) and basal (lower left frame). Cells to centre right (unframed) are late alveolar progenitors, while mature alveolar cells have been left out. Both are epithelial, they are the terminally differentiated cells that produce milk in the alveoli (especially the mature alveolar cells) and these are massively lost during post-lactational involution due to apoptosis, along with a large part of the ductal arborizations; **C.** Dot plots showing the distribution of luminal and basal cells according to the EpCAM and CD49f markers. The decrease in the luminal population is observed in lactating (i.e. breastfeeding) multiparous mice (central panel) and in multiparous mice treated with cabergoline (lower panel), in comparison with nulliparous mice (upper panel) and a relative increase in the basal population; **D.** Kruskal-Wallis test and Wilcoxon test showing comparison between groups of the proportion of luminal cells; and **E.** Kruskal-Wallis test and Wilcoxon test showing comparison between groups of the proportion of basal cells. Within the epithelial recombinant cells (RFP+ EpCAM+), a reduction in the proportion of luminal cells and a relative increase in basal cells was observed.

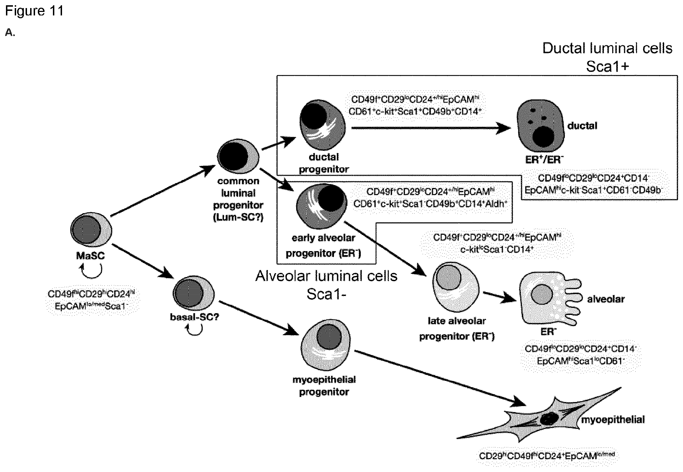

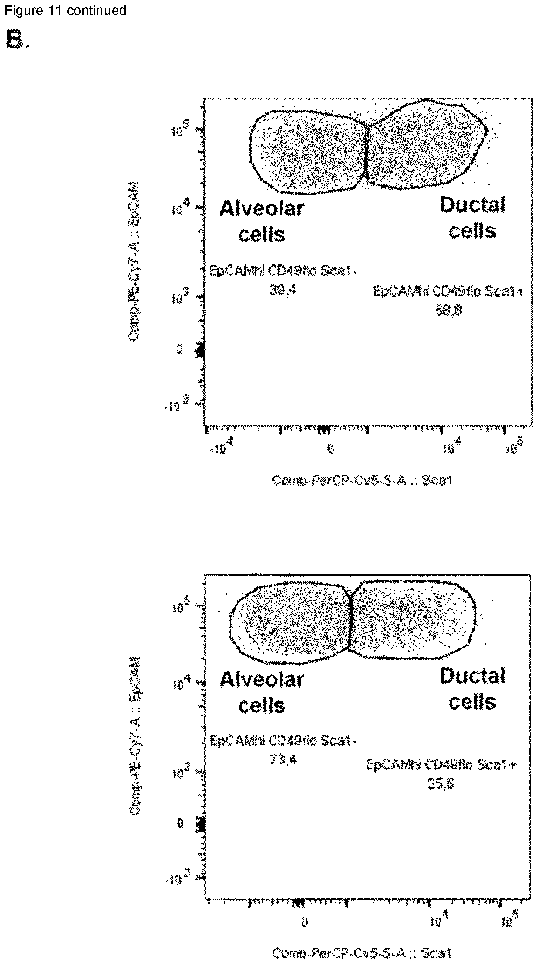

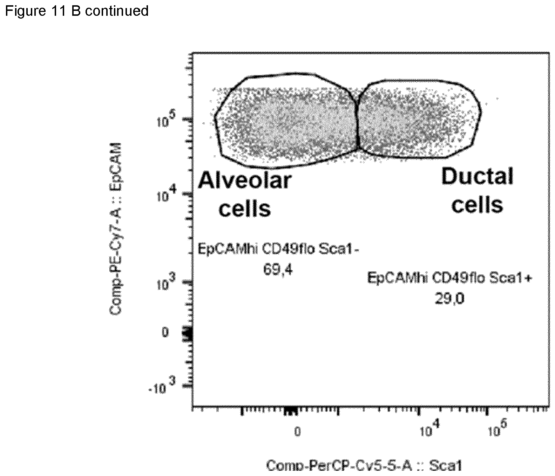

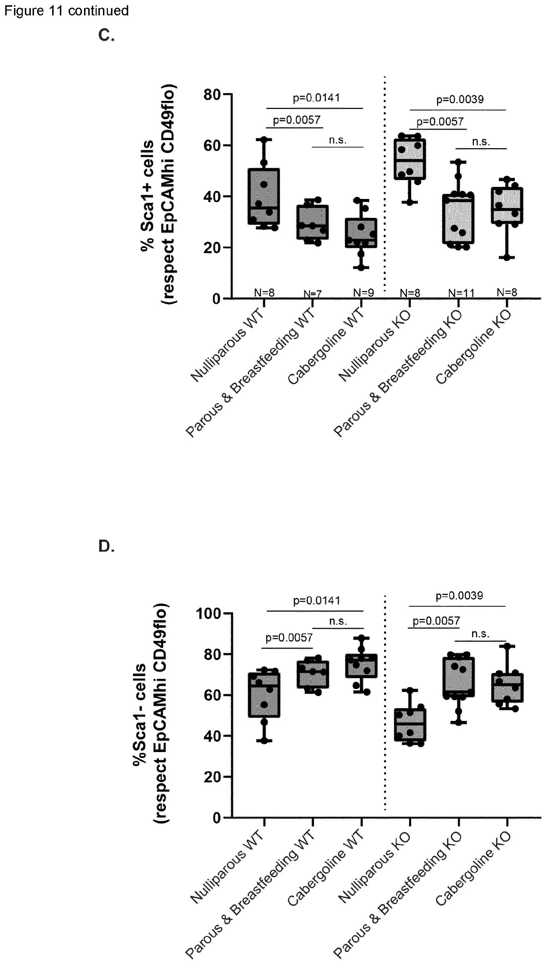

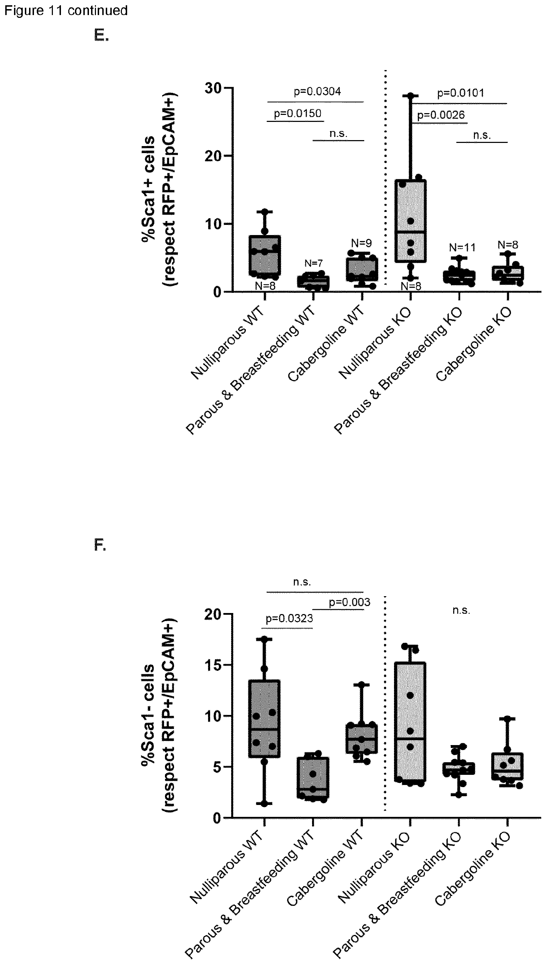

**Figure 11****.** Pregnancy produces a decrease in the ductal component (Sca1+) within the recombinant luminal cells, with no significant differences between the group treated with cabergoline. A. Diagram of the cellular hierarchy of the breast wherein ductal luminal populations (parent and mature, both Sca1+) and alveolar luminal precursors (Sca1-) are indicated. Only the alveolar progenitor (Sca1-) is framed separately; **B.** Dot plots showing the ductal and alveolar subpopulations within the total luminal (EpCAM hi/CD49f lo) recombinant (RFP+) cells. The decrease in the luminal ductal population is observed in multiparous mice not administered with cabergoline (center panel) and in multiparous mice administered with cabergoline (lower panel) but not in nulliparous mice (upper panel); Kruskal-Wallis test and Wilcoxon test showing comparison between groups of **C.** ductal cells and **D.** alveolar cells with respect to the total number of recombinant luminal cells (EpCAM hi/CD49f lo/RFP+); and Kruskal-Wallis test and Wilcoxon test showing comparison of the proportion of **E.** ductal cells and **F.** alveolar cells with respect to the total number of recombinant epithelial cells (RFP+ EpCAM+).

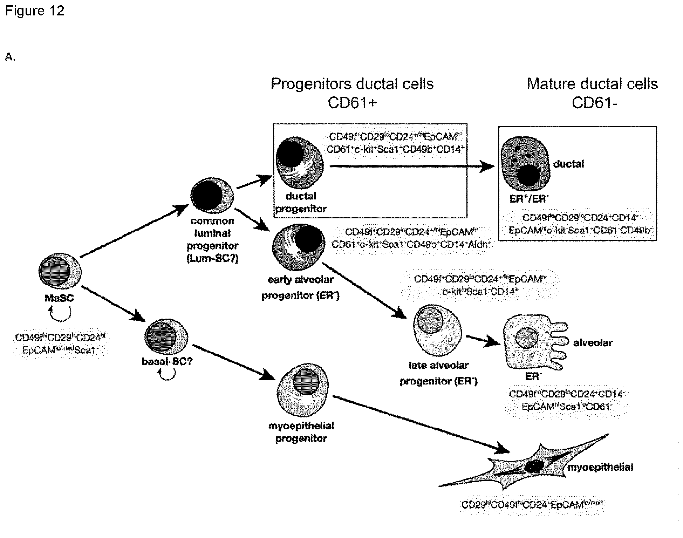

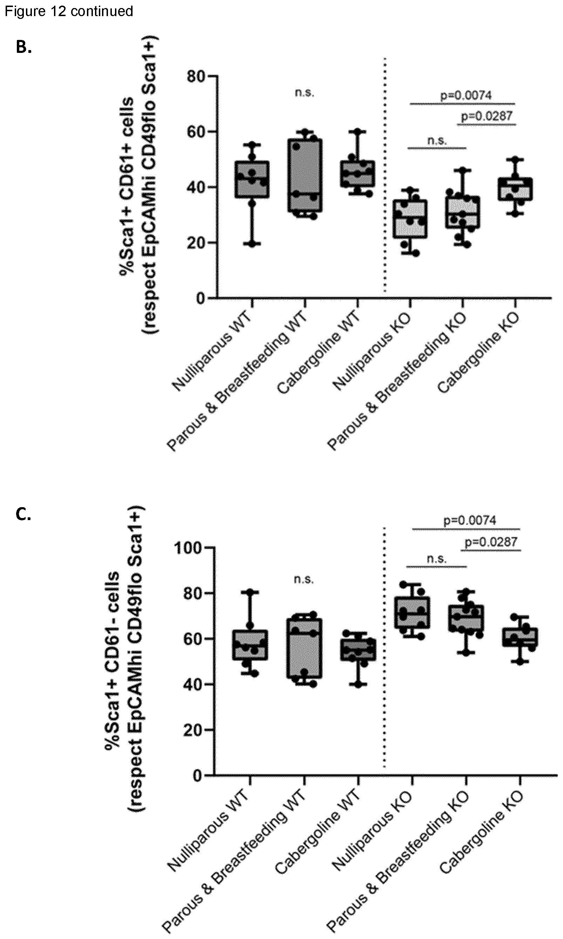

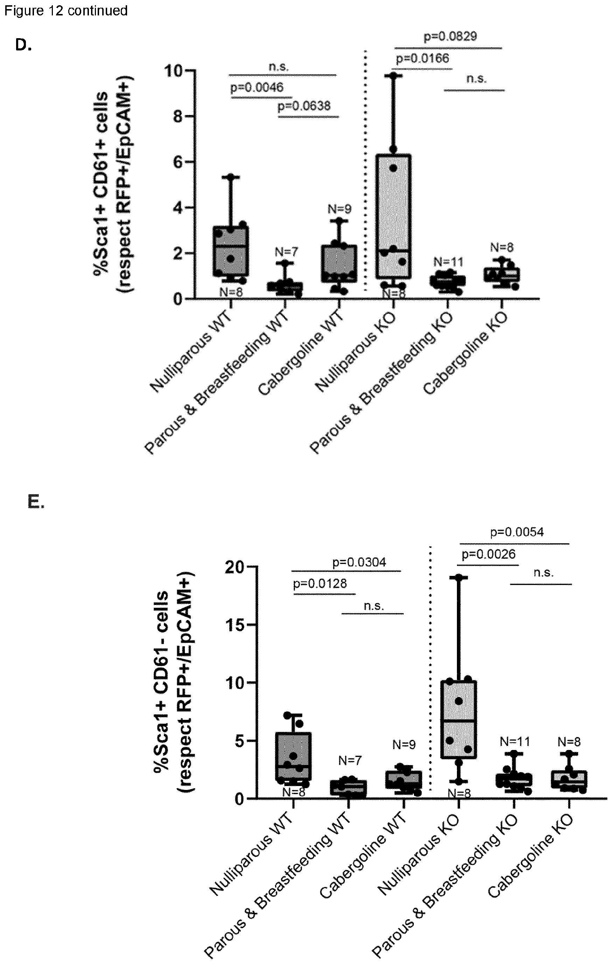

**Figure 12****.** Cabergoline induces a significant relative increase in luminal ductal progenitors within RFP+ recombinant cells, but without differences with respect to total recombinant cells. **A.** Diagram of the cellular hierarchy of the breast, wherein progenitor (CD61+) and mature (CD61-) ductal populations are indicated; Kruskal-Wallis test and Wilcoxon test showing comparison between groups of **B.** ductal precursor cells and **C.** mature ductal cells, with respect to the total number of recombinant ductal cells (EpCAM hi/CD49f Io/Sca1+/RFP+); and Kruskal-Wallis test and Wilcoxon test showing comparison of the proportion of **E.** ductal cells and **F.** alveolar cells with respect to the total of the recombinant epithelial cells (RFP+ EpCAM+).

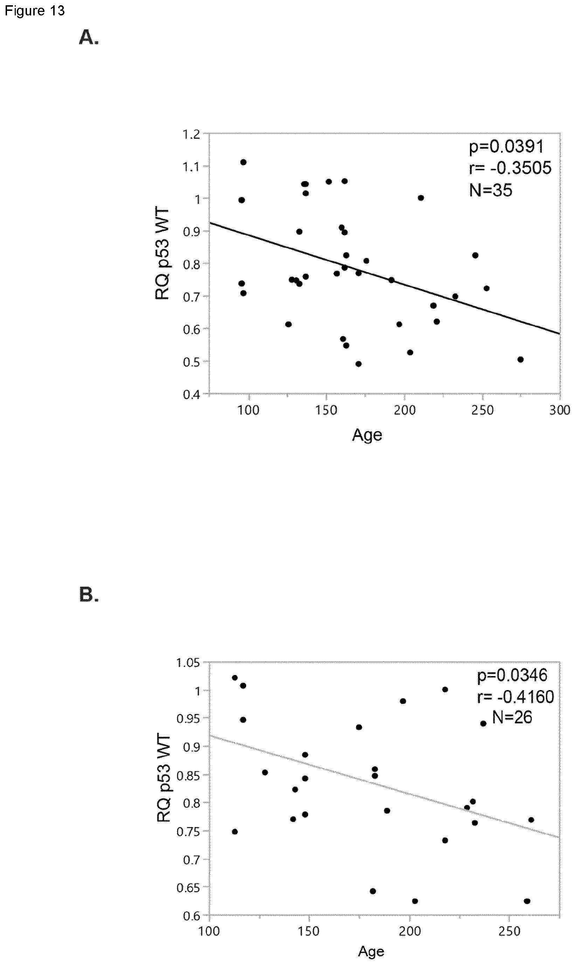

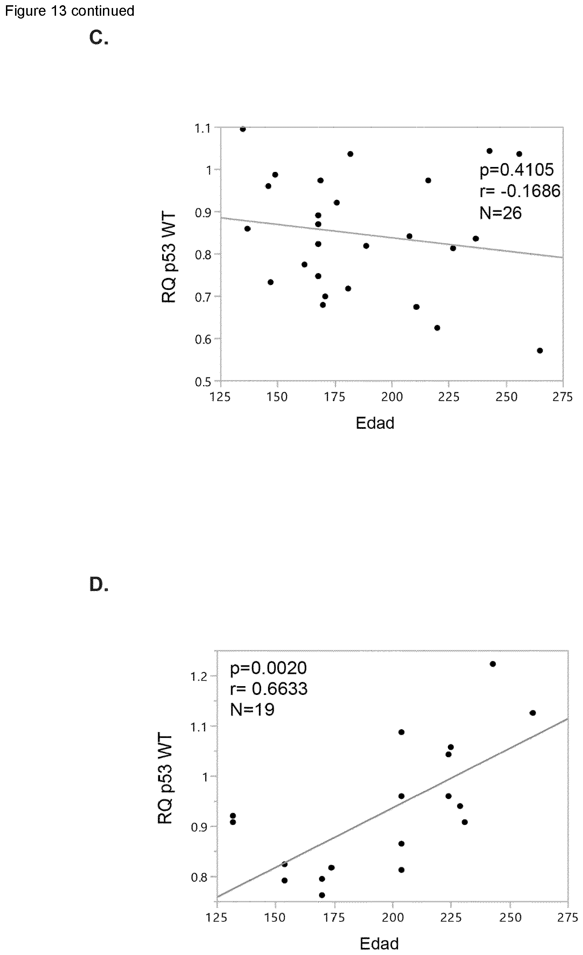

**Figure 13****.** Cabergoline produces a relative increase in the number of wild type (WT) cells in the breast determined by qPCR, which indirectly indicates a reduction in the number of recombinant cells. Quantification of the WT allele over time in the longitudinal cohort by qPCR in **A.** nulliparous mice; B. non-lactating (non-breastfeeding) multiparous mice; **C.** lactating (breastfeeding) multiparous mice; and D. lactating (breastfeeding) multiparous mice treated with cabergoline.

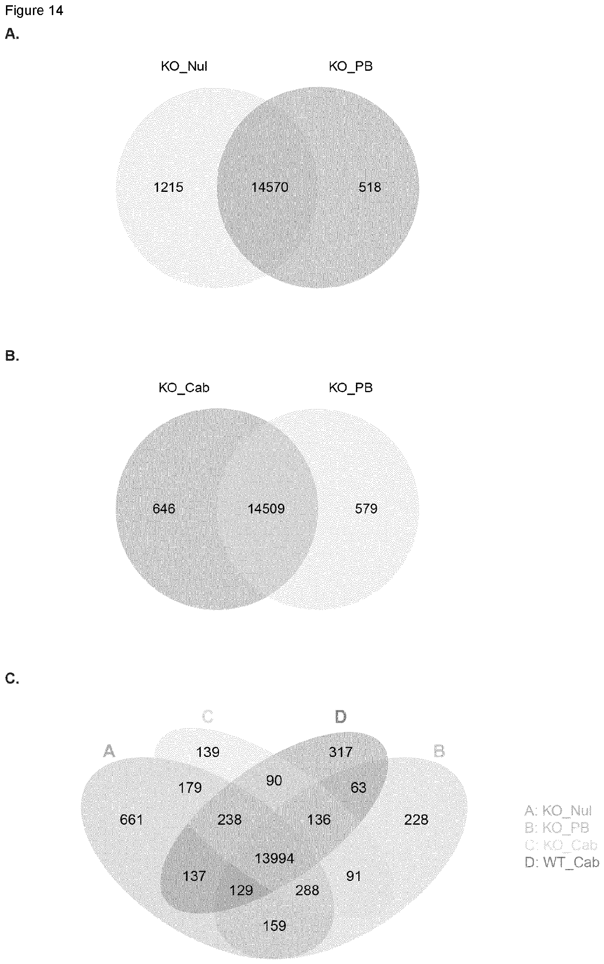

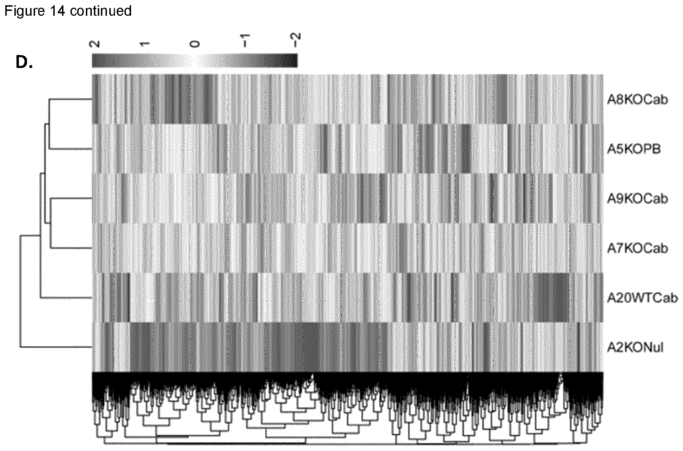

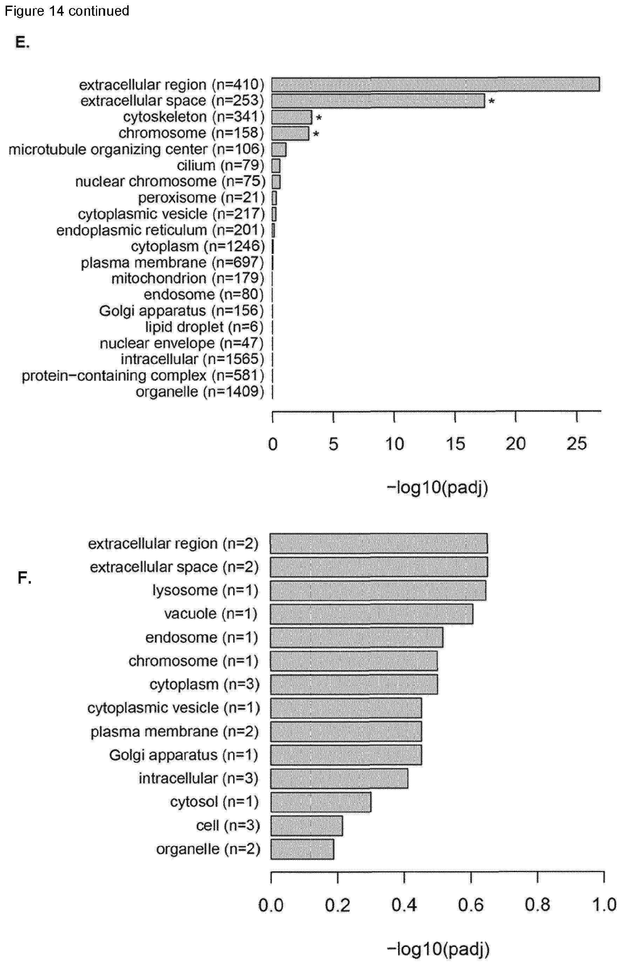

**Figure 14****.** Cabergoline modifies transcription. Venn diagrams of RNA-sequencing study on recombinant cells isolated by FACS sorting in **A.** sample obtained from a nulliparous knock-out (KO) mouse (KO_Nul) versus a sample obtained from a multiparous KO mouse without treatment (KO_PB) (PB, indicates "parous and breastfeeding"); **B.** sample obtained from a multiparous KO mouse without treatment (KO_PB) versus a sample obtained from a multiparous KO mouse treated with cabergoline; and C. sample obtained from a nulliparous KO mouse (KO_Nul) versus a sample obtained from a multiparous KO mouse without treatment (KO_PB) versus a sample obtained from a multiparous KO mouse treated with cabergoline (KO_Cab) versus a sample obtained from a multiparous wild-type mouse treated with cabergoline (WT_Cab); **D.** unsupervised cluster analysis of one sample obtained from a nulliparous KO mouse (A2KONul), one sample obtained from an untreated multiparous KO mouse (A5KOPB), 3 samples obtained from multiparous KO mice treated with cabergoline (A7KOCab, A8KOCab, A9KOCab), and one sample obtained from a multiparous WT mouse treated with cabergoline (A20WTCab)); E. gene ontology (GO) study in a sample obtained from a nulliparous KO mouse (KO_Nul) vs a sample obtained from a multiparous KO mouse without treatment (KO_PB); F. GO study between a sample obtained from a multiparous KO mouse without treatment (KO_PB) and a sample obtained from a multiparous KO mouse with treatment (KO_Cab).

### Detailed description of the invention

The present invention relates to cabergoline or a pharmaceutically acceptable salt or solvate thereof, for use in prevention or delay of post-pregnancy breast cancer in a female mammal. Analogously, the present invention relates to a method of prevention or delay of post-pregnancy breast cancer in a female mammal comprising administration thereto of cabergoline or a pharmaceutically acceptable salt or solvate thereof. Similarly, the present invention relates to the use of cabergoline or a pharmaceutically acceptable salt or solvate thereof for the manufacture of a medicament for prevention or delay of post-pregnancy breast cancer in a female mammal.

Cabergoline is a compound having the following chemical structure:

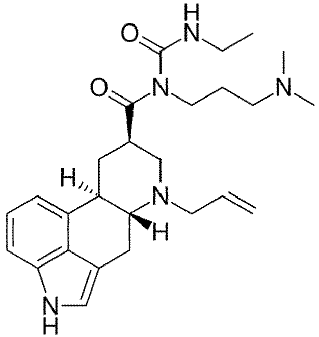

Cabergoline (CAS No: 81409-90-7) has the chemical name 1-[(6-allylergolin-8ß-yl)-carbonyl]-1-[3-(dimethylamino) propyl]-3-ethylurea [IUPAC nomenclature: (6*aR*,9*R*,10a*R*)-*N*-[3-(dimethylamino)propyl]-*N*-(ethylcarbamoyl)-7-prop-2-enyl-6,6*a*,8,9,10,10*a*-hexahydro-4*H*-indolo[4,3-fg]quinoline-9-carboxamide] and is sold under brand names including Dostinex^{®}, Galastop^{®}, Cabaser^{®} and Sogilen^{®}. Cabergoline is preferably apo-cabergoline (anhydrous, non-solvalted cabergoline. Even more preferably, said apo-cabergoline may exist as an amorphous solid or as one of four crystalline polymorphic forms. Cabergoline also exists as the diphosphate salt (CAS No: 85329-89-1).

Said solvate is preferably a pharmaceutically acceptable solvate. Said solvate may be a solvate of water (i.e. if crystalline, said co-crystal is a hydrate), a solvate of an organic solvent such as methanol, ethanol, dichloromethane, chloroform, acetone, acetonitrile or dimethylsulfoxide, a solvate or co-crystal of n-ethyl, n-propyl or n-butyl carboxylic acids. Said solvate is preferably a solvate of water. More preferably, said solvate is the monohydrate, dihydrate, trihydrate, tetrahydrate, pentahydrate or hexahydrate.

In the present invention, "prevention of post-pregnancy breast cancer" refers to preventing appearance or development of post-pregnancy breast cancer, or embodiments thereof, in said female mammal. On the other hand, in the present invention, "delay of post-pregnancy breast cancer" refers to delaying appearance or development of post-pregnancy breast cancer, or embodiments thereof, in said female mammal. Thus, delay of said post-pregnancy breast cancer may involve, for example, slowing the rate at which said post-pregnancy breast cancer appears or develops in said female mammal, wherein said rate may be slowed such that post-pregnancy breast cancer never appears or develops in said female mammal. In another example, delay of said post-pregnancy breast cancer may preferably involve delaying metastasis (and hence dissemination) of said post-pregnancy breast cancer.

In the present invention, any effect provided by pregnancy in prevention or delay of appearance or development of breast cancer in a female mammal is potentiated by cabergoline or a pharmaceutically acceptable salt or solvate thereof. Preferably said effect is synergic.

Said female mammal, as defined in the present invention, is preferably a female human or a female domesticated mammal such as a canine, feline, bovine, ovine, porcine, equine, camelline, caprine or cervine. More preferably, said mammal is a human (*i.e.* a woman), dog (i.e. a bitch), cattle (*i.e.* a cow), sheep (i.e. a ewe), pig (i.e. a sow), horse (i.e. a mare), or camel (i.e. a camel cow), even more preferably a human or dog. In a preferred embodiment of the present invention, said female mammal is a woman.

Said female mammal can be a breastfeeding/lactating female mammal. It is also contemplated that the female mammal is not breasfeeding/lactating. It may also be a female mammal that had an abortion after at least 34 weeks or more of gestation. The abortion may, for example, be due to fetal death.

In a preferred embodiment of the present invention, said female mammal expresses a dopamine D₂ receptor. Thus, said preferred embodiment of the present invention relates to cabergoline or a pharmaceutically acceptable salt or solvate thereof, for use in prevention or delay of post-pregnancy breast cancer in a female mammal, wherein said female mammal expresses a dopamine D₂ receptor. Analogously, a preferred embodiment of the method of the present invention relates to a method of prevention or delay of post-pregnancy breast cancer in a female mammal comprising administration thereto of cabergoline or a pharmaceutically acceptable salt or solvate thereof, wherein said female mammal expresses a dopamine D₂ receptor. Similarly, a preferred embodiment of the use of the present invention relates to use of cabergoline or a pharmaceutically acceptable salt or solvate thereof for the manufacture of a medicament for prevention or delay of post-pregnancy breast cancer in a female mammal, wherein said female mammal expresses a dopamine D₂ receptor. Said dopamine D₂ receptor is preferably that encoded by the *DRD2* gene when said female mammal is a woman.

In a preferred embodiment of the present invention, said female mammal expresses a dopamine D₂ receptor, and/or a dopamine D₁ receptor, and/or a α1-adrenergic receptor, and/or a α2- adrenergic receptor, and/or a 5-HT1 serotonin receptor, and/or a 5-HT2-serotonin receptor. Thus, said preferred embodiment of the present invention relates to cabergoline or a pharmaceutically acceptable salt or solvate thereof, for use in prevention or delay of post-pregnancy breast cancer in a female mammal, wherein said female mammal expresses a dopamine D₂ receptor, and/or a dopamine D₁ receptor, and/or a α1-adrenergic receptor, and/or a α2- adrenergic receptor, and/or a 5-HT1 serotonin receptor, and/or a 5-HT2-serotonin receptor. Analogously, a preferred embodiment of the method of the present invention relates to a method of prevention or delay of post-pregnancy breast cancer in a female mammal comprising administration thereto of cabergoline or a pharmaceutically acceptable salt or solvate thereof, wherein said female mammal expresses a a dopamine D₂ receptor, and/or a dopamine D₁ receptor, and/or a α1-adrenergic receptor, and/or a α2- adrenergic receptor, and/or a 5-HT1 serotonin receptor, and/or a 5-HT2-serotonin receptor. Similarly, a preferred embodiment of the use of the present invention relates to use of cabergoline or a pharmaceutically acceptable salt or solvate thereof for the manufacture of a medicament for prevention or delay of post-pregnancy breast cancer in a female mammal, wherein said female mammal expresses a a dopamine D₂ receptor, and/or a dopamine D₁ receptor, and/or a α1-adrenergic receptor, and/or a α2- adrenergic receptor, and/or a 5-HT1 serotonin receptor, and/or a 5-HT2-serotonin receptor.

Said dopamine D₂ receptor is preferably the one encoded by the *DRD2* gene when said female mammal is a woman. Said dopamine D₁ receptor is also preferably the one encoded by the *DRD1* gene when said female mammal is a woman. Said α1-adrenergic receptor is preferably the one encoded by the *ADRA1A* (adrenoceptor alpha 1A) gene when said female mammal is a woman. Said α2-adrenergic receptor is also preferably the one encoded by the *ADRA2A* (alpha-2-adrenergic receptors) gene when said female mammal is a woman. Said 5-HT1 serotonin receptor is preferably the one encoded by the *HTR1A* (5-hydroxytryptamine receptor 1A) gene when said female mammal is a woman. Said 5-HT2 serotonin receptor is also preferably the one encoded by the *HTR2A* (Serotonin 5-HT2A receptors) gene when said female mammal is a woman.

Preferably, said female mammal has a mutation in a tumour suppressor gene and/or an oncogene. Preferably, said tumor suppressor gene is a breast cancer suppressor gene selected from the group consisting of: *Brca1, Brca2, P53 (TP53), CHK2, FANCD2, ATM, BRIP1, PALB2* (*FANCN*), *PTEN and STK11* and said oncogene is an epidermal growth factor receptor gene such as the *ErbB2* (*HER2*/*neu*) gene. More preferably, said female mammal has a mutation in the *Brca1* gene, *Brca2* gene and/or the *P53* gene and/or the *ErbB2* gene, even more preferably a mutation in the *Brca1* gene and/or the *P53* gene and/or the *ErbB2* gene. In a preferred embodiment of the present invention, said female mammal has a mutation in the *Brca1* gene and/orthe *P53* gene. Thus, said preferred embodiment of the present invention relates to cabergoline or a pharmaceutically acceptable salt or solvate thereof, for use in prevention or delay of post-pregnancy breast cancer in a female mammal, wherein said female mammal has a mutation in the *Brca1* gene and/or the *P53* gene. Analogously, a preferred embodiment of the method of the present invention relates to a method of prevention or delay of post-pregnancy breast cancer in a female mammal comprising administration thereto of cabergoline or a pharmaceutically acceptable salt or solvate thereof, wherein said female mammal has a mutation in the *Brca1* gene and/or the *P53* gene. Similarly, a preferred embodiment of the use of the present invention relates to use of cabergoline or a pharmaceutically acceptable salt or solvate thereof for the manufacture of a medicament for prevention or delay of post-pregnancy breast cancer in a female mammal, wherein said female mammal has a mutation in the *Brca1* gene and/or the *P53* gene.

Thus, said breast cancer (including post-pregnancy breast cancer), as defined herein, may be either hereditary breast cancer or non-hereditary breast cancer. An example of hereditary breast cancer includes that caused by a mutation in the *Brca1* gene and/or the *P53* gene, while an example of non-hereditary breast cancer includes that caused by a mutation in the *ErbB2* gene.

Said post-pregnancy breast cancer, as defined in the present invention, is any breast cancer (breast tumour) that appears or develops following pregnancy in a female mammal. Thus, said post-pregnancy breast cancer, as defined in the present invention, is a proliferative disease of breast tissue, preferably a cancer (tumour) of the breast tissue which exhibits anaplasia, invasiveness and/or metastasis. In a preferred embodiment of the present invention, said breast cancer is metastatic. Thus, said preferred embodiment of the present invention relates to cabergoline or a pharmaceutically acceptable salt or solvate thereof, for use in prevention or delay of metastatic post-pregnancy breast cancer in a female mammal. Analogously, a preferred embodiment of the method of the present invention relates to a method of prevention or delay of metastatic post-pregnancy breast cancer in a female mammal comprising administration thereto of cabergoline or a pharmaceutically acceptable salt or solvate thereof. Similarly, a preferred embodiment of the use of the present invention relates to use of cabergoline or a pharmaceutically acceptable salt or solvate thereof for the manufacture of a medicament for prevention or delay of metastatic post-pregnancy breast cancer in a female mammal. Even more preferably, said post-pregnancy breast cancer, as defined in the present invention, is a breast adenocarcinoma.

Said post-pregnancy breast cancer is moreover selected from a cancer of the epithelial or mesenchymal tissues of the breast. Preferably, said post-pregnancy breast cancer is a cancer of epithelial tissue of the breast. In a preferred embodiment of the present invention, said post-pregnancy breast cancer is a cancer of ductal epithelial tissue. Thus, said preferred embodiment of the present invention relates to cabergoline or a pharmaceutically acceptable salt or solvate thereof, for use in prevention or delay of ductal epithelial post-pregnancy breast cancer in a female mammal. Analogously, a preferred embodiment of the method of the present invention relates to a method of prevention or delay of ductal epithelial post-pregnancy breast cancer in a female mammal comprising administration thereto of cabergoline or a pharmaceutically acceptable salt or solvate thereof. Similarly, a preferred embodiment of the use of the present invention relates to use of cabergoline or a pharmaceutically acceptable salt or solvate thereof for the manufacture of a medicament for prevention or delay of ductal epithelial post-pregnancy breast cancer in a female mammal. Even more preferably, said post-pregnancy breast cancer, as defined in the present invention, is a cancer of luminal ductal epithelial tissue.

As defined above, said post-pregnancy breast cancer is any breast cancer that is prevented or delayed from appearing or developing following pregnancy (i.e. not prior to any pregnancy or during pregnancy) in a female mammal. However, when said female mammal is a parous woman, said post-pregnancy breast cancer preferably is that which is prevented or delayed up to 20 years after having given birth, more preferably up to 10 years after having given birth, even more preferably between 2 and 8 years after having given birth, still more preferably between 4 and 7 years after having given birth. Thus, in the embodiment of the present invention relating to cabergoline or a pharmaceutically acceptable salt or solvate thereof, for use in prevention or delay of post-pregnancy breast cancer in a female mammal, wherein said female mammal is a parous woman, said post-pregnancy breast cancer preferably is that which is prevented or delayed up to 20 years after having given birth, more preferably up to 10 years after having given birth, even more preferably between 2 and 8 years after having given birth, still more preferably between 4 and 7 years after having given birth. Analogously, in the embodiment of the present invention relating to a method of prevention or delay of post-pregnancy breast cancer in a female mammal comprising administration thereto of cabergoline or a pharmaceutically acceptable salt or solvate thereof, wherein said female mammal is a parous woman, said post-pregnancy breast cancer preferably is that which is prevented or delayed up to 20 years after having given birth, more preferably up to 10 years after having given birth, even more preferably between 2 and 8 years after having given birth, still more preferably between 4 and 7 years after having given birth. Similarly, in the embodiment of the present invention relating to use of cabergoline or a pharmaceutically acceptable salt or solvate thereof for the manufacture of a medicament for prevention or delay of ductal epithelial post-pregnancy breast cancer in a female mammal, wherein said female mammal is a parous woman, said post-pregnancy breast cancer preferably is that which is prevented or delayed up to 20 years after having given birth, more preferably up to 10 years after having given birth, even more preferably between 2 and 8 years after having given birth, still more preferably between 4 and 7 years after having given birth.

Said post-pregnancy breast cancer is any breast cancer that is prevented or delayed from appearing or developing following a first, second, third, fourth, fifth or more pregnancies (and hence births), preferably following a first, second, third or fourth pregnancies, more preferably following first, second or third pregnancies.

Said post-pregnancy breast cancer preferably may be any breast cancer that is prevented or delayed from appearing or developing following weaning of the last offspring to have been born from the last pregnancy. More preferably, said post-pregnancy breast cancer may be any breast cancer that is prevented or delayed from appearing or developing in a female mammal who has weaned their offspring. Thus, a preferred embodiment of the present invention relates to cabergoline or a pharmaceutically acceptable salt or solvate thereof, for use in prevention or delay of post-pregnancy breast cancer in a female mammal, wherein said female mammal has weaned their offspring. Analogously, a preferred embodiment of the method of the present invention relates to a method of prevention or delay of post-pregnancy breast cancer in a female mammal comprising administration thereto of cabergoline or a pharmaceutically acceptable salt or solvate thereof, wherein said female mammal has weaned their offspring. Similarly, a preferred embodiment of the use of the present invention relates to use of cabergoline or a pharmaceutically acceptable salt or solvate thereof for the manufacture of a medicament for prevention or delay of post-pregnancy breast cancer in a female mammal, wherein said female mammal has weaned their offspring.

Even more preferably, said post-pregnancy breast cancer may be that which is prevented or delayed from appearing or developing once lactation or milk production has begun to cease or has fully ceased (i..e. weaning has begun/breastfeeding has ceased). Still more preferably, said post-pregnancy breast cancer may be that which is prevented or delayed from appearing or developing during or after post-lactational involution. Thus, a preferred embodiment of the present invention relates to cabergoline or a pharmaceutically acceptable salt or solvate thereof, for use in prevention or delay of post-pregnancy breast cancer in a female mammal, wherein said cabergoline or said salt or solvate is administered to said female mammal during and/or after post-lactational involution, more preferably during post-lactational involution. Analogously, a preferred embodiment of the method of the present invention relates to a method of prevention or delay of post-pregnancy breast cancer in a female mammal comprising administration thereto of cabergoline or a pharmaceutically acceptable salt or solvate thereof, wherein said cabergoline or said salt or solvate is administered to said female mammal during and/or after post-lactational involution, more preferably during post-lactational involution. Similarly, a preferred embodiment of the use of the present invention relates to use of cabergoline or a pharmaceutically acceptable salt or solvate thereof for the manufacture of a medicament for prevention or delay of post-pregnancy breast cancer in a female mammal, wherein said cabergoline or said salt or solvate is administered to said female mammal during and/or after post-lactational involution, more preferably during post-lactational involution.

When said female mammal is a parous woman, said woman is preferably ≥ 25 years old when having given birth, more preferably when having first given birth. In a preferred embodiment of the present invention, said woman is ≥ 30 years old when having given birth, more preferably said woman is ≥ 35 years old when having given birth, even more preferably wherein "having given birth" means "having first given birth". Thus, said preferred embodiment of the present invention relates to cabergoline or a pharmaceutically acceptable salt or solvate thereof, for use in prevention or delay of post-pregnancy breast cancer in a woman, wherein said woman is ≥ 30 years old when having given birth, more preferably ≥ 35 years old when having given birth, even more preferably wherein "having given birth" means "having first given birth". Analogously, a preferred embodiment of the method of the present invention relates to a method of prevention or delay of post-pregnancy breast cancer in a female mammal comprising administration thereto of cabergoline or a pharmaceutically acceptable salt or solvate thereof, wherein said woman is ≥ 30 years old when having given birth, more preferably ≥ 35 years old when having given birth, even more preferably wherein "having given birth" means "having first given birth". Similarly, a preferred embodiment of the use of the present invention relates to use of cabergoline or a pharmaceutically acceptable salt or solvate thereof for the manufacture of a medicament for prevention or delay of post-pregnancy breast cancer in a female mammal, wherein said woman is ≥ 30 years old when having given birth, more preferably ≥ 35 years old when having given birth, even more preferably wherein "having given birth" means "having first given birth".

In another embodiment when said female mammal is a parous woman, said woman was preferably pregnant for more than 32 weeks during at least one pregnancy, more preferably during each pregnancy. In a preferred embodiment of the present invention, said female mammal is a parous woman who was pregnant for more than 34 weeks. Thus, said preferred embodiment of the present invention relates to cabergoline or a pharmaceutically acceptable salt or solvate thereof, for use in prevention or delay of post-pregnancy breast cancer in a woman, wherein said woman was pregnant for more than 34 weeks. Analogously, a preferred embodiment of the method of the present invention relates to a method of prevention or delay of post-pregnancy breast cancer in a female mammal comprising administration thereto of cabergoline or a pharmaceutically acceptable salt or solvate thereof, wherein said woman was pregnant for more than 34 weeks. Similarly, a preferred embodiment of the use of the present invention relates to use of cabergoline or a pharmaceutically acceptable salt or solvate thereof for the manufacture of a medicament for prevention or delay of post-pregnancy breast cancer in a female mammal, wherein said woman was pregnant for more than 34 weeks. More preferably, when said female mammal is a parous woman, said woman was preferably pregnant for more than 36 weeks during at least one pregnancy, yet more preferably during each pregnancy.

Even more preferably, said post-pregnancy breast cancer may be that which is prevented or delayed from appearing or developing in a female mammal such as a woman with at least 34 weeks of gestation. Thus, the female mammal such as a woman may have been pregnant for at least 34 weeks of gestation (34 weeks post-gestation). Said post-pregnancy breast cancer may be that which is prevented or delayed from appearing or developing in a female mammal such as a woman that has been pregnant for at least 34 weeks, at least 35 weeks, 36 weeks, 37 weeks, 38 weeks, 39 weeks, 40 weeks, 41 weeks, 42 weeks, 43 weeks, 44 weeks, 45 weeks or more of gestation. Said post-pregnancy breast cancer may be that which is prevented or delayed from appearing or developing in a female mammal, such as a woman that has been pregnant for at least 35-44 weeks, 36-43 weeks, 37-41 weeks of gestation. Thus, a preferred embodiment of the present invention relates to cabergoline or a pharmaceutically acceptable salt or solvate thereof, for use in prevention or delay of post-pregnancy breast cancer in a female mammal, wherein said cabergoline or said salt or solvate is administered to said female mammal during and/or after at least 35 weeks of gestation. Analogously, a preferred embodiment of the method of the present invention relates to a method of prevention or delay of post-pregnancy breast cancer in a female mammal comprising administration thereto of cabergoline or a pharmaceutically acceptable salt or solvate thereof, wherein said cabergoline or said salt or solvate is administered to said female mammal such during and/or after at least 35 weeks of gestation. Similarly, a preferred embodiment of the use of the present invention relates to use of cabergoline or a pharmaceutically acceptable salt or solvate thereof for the manufacture of a medicament for prevention or delay of post-pregnancy breast cancer in a female mammal, wherein said cabergoline or said salt or solvate is administered to said female mammal during and/or after at least 34 weeks of gestation.

As used herein gestation is a determinant of organ maturity. The gestational age can be calculated from the beginning on the first day of the female mammal's, such as a woman's, last menstrual period. If the conception day is known the gestational age may also be detemerined by the difference between 14 days before the date of conception and the day of delivery. A normal pregnancy can range from 38 to 42 weeks. Usually the female mammal such as a woman can tell the first day of the last menstrual period or even the date of cenception. When the date of conception is unknown and the menstrual cycles are irregular, unreliable, or information about them is not available, the gestational age may also be determined by any other suitable mean. For example, the gestational age may be determined by ultrasonographic measurements of the fetus. Preferably such ultrasonic measurements are conducted in the first trimester. How such ultrasonic measurements affect the redating of gestational age is described by American College of Obstetricians and Gynecologists' Committee on Obstetric Practice (2017) Methods for Estimating the Due Date, Committee Opinion, Number 700.

### Examples

### Materials and methods

### Mice. Study model

The study model used was a genetically modified mouse, generated by the group led by Jos Jinkers from the *Netherland Cancer Institute* and generously donated to our group. It is a conditional double knock-out (KO) for *Brca1* and *P53* that expresses Cre-recombinase under the promoter of the cytokeratin-14 gene *(K14)* as a model named: *K14Cre*; *Brca1*^{*F*/*F*}; *P53*^{*F*/*F*} (hereinafter *Brca1* or KO). These mice develop mammary tumours (cancers) with a penetrance of 100% (although it depends on whether they develop skin cancer first and must be sacrificed) and a median age of 7 months. They mimic human tumours with *BRCA1* mutations, since they are basal-type and lose *P53.* Since Cre-recombinase under the promoter of the K14 gene is not only expressed in mammary cells, but also in other epithelial cells, these mice develop squamous cell carcinoma-like skin tumors. The cohort of mice used in this study has an FVB/N genetic background.

Mice were divided into groups: (i) nulliparous N = 35; (ii) non-lactating multiparous (two pregnancies, not followed by breastfeeding) N = 33; (iii) lactating multiparous (= parous and breastfeeding) (two pregnancies, followed by breastfeeding) N = 32; and (iv) lactating multiparous and cabergoline (two pregnancies, followed by breastfeeding and the administration of an intraperitoneal dose of cabergoline of 0.25 mg/Kg at the time of weaning, after the second period of lactation/breastfeeding) N = 37.

Subsequently, the same groups of animals indicated were generated and samples were collected from the mammary glands at 1, 3, 7, 30 and 60 days post-gestation (in non-lactating multiparous) or post-weaning (in lactating multiparous, with and without treatment with cabergoline) (N = 5 mice per group at each time studied).

The mouse model used for the cytometry studies was obtained by crossing the conditional model *K14Cre*; *Brca1*^{*F*/*F*}; *P53*^{*F*/*F*} with a model that expresses red fluorescent protein (RFP) under the promoter of the ROSA26 locus. This promoter has a stop codon flanked by *LoxP* sites that recognize Cre-recombinase. In this way, cells that lose *Brca1* (gene) and *P53* (gene) due to the action of Cre-recombinase also express RFP and emit fluorescence. The mouse was obtained from the European repository (European mutant mouse archive - EMMA) and was generated by the group of Hans Jörg Fehling from the Institute of Immunology of the Clinical University of Ulm, Germany. For these studies, between 6 and 11 mice from the different study groups were used.

### Evaluation of pathophenotypes of breast cancer

Different pathophenotypes were considered to characterize susceptibility to breast cancer in the *Brca1* KO mouse cohort under the different study conditions. These were: (i) tumour latency, which is defined as the time from birth of the mouse to the appearance of the first mammary tumour; (ii) the tumour incidence, which is the percentage of mice that develop a mammary tumour with respect to the total of that group. To evaluate these parameters, the females deficient in *Brca1* and *p53* from the different study groups were checked and palpated once a week. The animals were sacrificed when they showed signs of disease or the tumours reached 2.5 cm in their greatest diameter. The study was approved by the Bioethics Committee of the University of Salamanca.

### Embedding of tissues in paraffin

The left inguinal breast of each mouse was embedded in paraffin for further study. Once removed from the animals, the breasts were fixed in 4% paraformaldehyde (Scharlau FO) for 24 hours and, subsequently, they were kept in 70% ethanol at a temperature of 4 °C, until they were processed in the Comparative Molecular Pathology Service of the Salamanca Cancer Research Center. Tissues were dehydrated by three 1-hour incubations in increasing concentrations of ethanol (60%, 80%, and 100%). Three other 1-hour incubations were carried out in xylol, to replace the dehydrating agent with a substance miscible in paraffin. Finally, three other incubations of 1 hour and 20 minutes were carried out with liquid paraffin, after which blocks were made with a mould suitable for the size of the tissue in a paraffin embedding station. When the paraffin blocks were completely cold, 2-µm-thick sections were made using a rotation microtome (Leica RM2255).

### Hematoxylin-eosin staining

Hematoxylin and eosin staining was performed in the Comparative Molecular Pathology Service of the Salamanca Cancer Research Center using a battery of manual staining following the processes: deparaffinization in xylol, rehydration with ethanol at different graduations, hematoxylin and eosin staining, dehydration with ethanol and clearance. Once the process was finished, the samples were mounted by adding a drop of non-aqueous DPX fixative and the coverslip was placed.

### Caspase 3 detection with immunohistochemistry as a marker of apoptosis

Sections were deparaffinized and rehydrated. Antigen unmasking was then performed with the Antigen Unmasking Solution (Vector Labs #H-3300). After heating the slide for 20 minutes in the microwave, endogenous peroxidases were blocked with Blocking BLOXALL Solution (#SP-6000). Afterwards, the sections were washed with PBS 1X (0.137 M NaCl, 0.0027 M KCI, 0.01 M Na₂HPO₄, 0.0018 M KH₂PO₄; the concentracions as used in the experiment) and the possible nonspecific binding sites of the primary antibody were blocked by incubation for 30 minutes with fetal bovine serum diluted in PBS. After blocking, samples were incubated overnight at 4 °C with a 1:300 dilution of non-activated caspase 3 primary antibody (produced in rabbit) diluted in PBS 1X (concentration as used in the experiment) with 0.1% Triton X-100 and 5% rabbit serum. (#9661, Cell Signaling). The next day, the samples were washed with PBS and incubated with the secondary antibody (anti-rabbit) for 30 minutes. For development, the Vectastain elite ABC Kit (Vector Labs #PK-6100) was used, which uses diaminobenzidine (DAB) as a chromogen. Finally, a hematoxylin counterstain was performed and the samples were dehydrated and cleared. The equipment used to carry out the immunohistochemistry was the Discovery Ultra (Roche).

### Detection of Ki-67 by immunohistochemistry as a proliferation marker

Ki67 staining was performed at the Comparative Molecular Pathology Service of the Cancer Molecular and Cellular Biology Institute in Salamanca. Sections of the sample were made at 3 µm thickness. Sections were fished onto slides specially treated for this technique (Leica BOND Plus Slides, #S21.2113.A, Leica). The staining process was carried out using the Roche Discovery ULTRA research instrument. The protocol performed by the apparatus is as follows: deparaffinization of the sample, heating the slide to 69 °C; cell conditioning that consisted in the unmasking of the epitope by incubating the sample in a buffer formed by TRIS EDTA pH8 at a temperature of 100 °C for 32 minutes. Next, the primary antibody (Rabbit Anti-Human Ki-67 Monoclonal Antibody (Clone SP6), #MAD-020310Q, Master Diagnostica) was applied manually at a 1:50 dilution and incubated at 37 °C for 60 minutes. After incubation of the primary antibody, two washes with HRP buffer were performed. Subsequently, the sample was incubated with the prediluted secondary antibody (OmniMap anti-Rb HRP, #05269679001, Roche) conjugated with horseradish peroxidase for 16 minutes and revealed with diaminobenzidine (DAB). Finally, a hematoxylin counterstain was performed and the slide was cleaned. To finish, the coverslip was manually mounted by applying a drop of DPX resin.

### Quantification of adipose and ductal tissue surfaces in the breast

Adipose tissue area and ductal area were evaluated using ImageJ free software. The ductal area was defined as the surface occupied by the epithelium of the ducts together with the surrounding fibrous tissue stroma, excluding the area of the tubular lumen. To evaluate the ductal and adipocyte area, 10 photos were taken at 10X magnification of the surface of the mammary gland of each of the study animals, five photos in the distal area and five in the proximal area of the breast. The photos were taken randomly in each of these areas of the samples stained with hematoxylin-eosin, with a Leica DM1000 light microscope. The evaluation of the percentages of ductal area and adipose tissue area showed a gradient from greater to lesser involution from the proximal to the distal area. In the proximal area, more adipose tissue and less ductal tissue were observed than in the distal area, and *vice versa.* For this reason, only the values of the distal zone were determined to carry out the analyses.

The quantification was expressed as a percentage of the total area. The percentages of the ductal area and the area of adipocytes in the breast were defined as the total of the ductal surface and the surface occupied by adipocytes divided, respectively, by the total area of the mammary gland present in each photo (multiplied by one hundred to express them as percentages).

### Quantification of positive cells in immunohistochemical reactions

To quantify cells positive for Ki-67 or caspase 3, the Leica Application Suite (LAS) V3.7 program was used. Eight photos with 20X magnification of the mammary gland were taken with a Leica DM1000 light microscope. Next, the parameters were defined for the program to distinguish positive from negative cells. To analyse each photo, the portion of interest (ductal epithelium) was manually defined, then the program automatically provided the number of positive and negative cells in the selected region. In both cases, the percentage of positive cells was defined as the number of positive cells divided by the number of total cells (positive plus negative).

### Preparation of organoids from breast epithelial tissue

In order to obtain a suspension enriched in mouse mammary epithelial cells, from which we can obtain both RNA and proteins and also maintain acceptable viability, the protocol for obtaining mouse mammary organoids was carried out. Once the mammary glands were removed after performing the necropsy of the mouse, they were mechanically disintegrated by cutting with two scalpels. Subsequently, they were incubated in a volume of 10 mL of supplemented medium (DMEM/F12 (50%/50%), 5% fetal bovine serum, 1% penicillin/streptomycin) plus 1.25 mL of collagenase, and 1 mL of trypsin. This enzymatic digestion was carried out for 30 minutes at 37 °C in orbital agitation at 100 revolutions per minute (rpm) (= 1.666 s⁻¹). After incubation, the sample was centrifuged for 5 minutes at 1200 rpm (= 20 s⁻¹) at room temperature. The product of the centrifugation was three phases, the upper one formed by the fat of the breasts, the intermediate aqueous one and the pellet where the organoids are found. The first two phases were absorbed, leaving us with the pellet. To the pellet obtained, 4 mL of PBS supplemented with 2% foetal bovine serum and 119 µL of DNase (20 U/mL) were added. The pellet was mixed by gently shaking it and incubated for 5 minutes at room temperature. Subsequently, it was centrifuged for 5 minutes at 1200 rpm (= 20 s⁻¹) at room temperature, once the centrifugation was finished, the supernatant was aspirated and three washes were performed with 4 mL of PBS supplemented with cold 2% foetal bovine serum, giving a pulse of 1500 rpm (= 25 s⁻¹) for 5 seconds and decanting the supernatant between washes. Finally, 1 mL of cold supplemented PBS was added and divided into two RNase-free 1.5 mL tubes, which were centrifuged for 5 minutes at 2200 rpm (= 36.666 s⁻¹) at a temperature of 4 °C. After centrifugation, the supernatant was removed with a P200 pipette and the pellet was frozen in liquid nitrogen.

### Indirect estimation of the number of recombinant P53 cells by quantitative PCR

DNA was extracted from frozen organoids obtained from mammary glands at 30 and 60 days of involution, and from tumour-free mammary glands of mice that developed breast cancer over time. The quantity and quality of DNA were quantified using a spectrophotometer (Nanodrop). Subsequently, this DNA was used to determine the recombination frequency of the *P53* gene in the breasts of the different groups by Quantitative Polymerase Chain Reaction (qPCR). TaqMan probes were used, one directed against the sequence of exon 6 of *P53* (#PN4400291, Applied Biosystems) and the other against the *Tfrc* gene (transferrin receptor gene) (#4458370, Applied Biosystems), used as a reference gene.

For the qPCR reaction, 2 µL of template DNA (10 ng), 10 µL of the Applied Genotyping Master Mix buffer, 1 µL of TaqMan probe and 7 µL of waterwere mixed. The program consisted of two incubations, one at 50 °C for two minutes and the other at 95 °C for 15 seconds, followed by amplification for 40 cycles. Each of these cycles consisted of a DNA denaturation phase at 95 °C for 15 seconds and a subsequent phase at 60 °C for one minute.

For each sample, qPCR reactions against *P53* and *Tfrc* were performed in parallel and in triplicate. The determinations were carried out in 96-well Twin.tec real-time PCR plates (#0030132513, Eppendorf), hermetically covered with heat sealing film adhesives (#0030127838, Eppendorf). The qPCR reactions and the reading of the signal were performed in the Mastercycler epgradient S Realplex2 thermal cycler from Eppendor.

For data analysis, the 2^{-ΔΔCt} method was used to estimate the relative quantity (Relative Quantity or RQ) of recombined cells from each sample. In each reaction, the limit cycle or Ct (cycle threshold) was obtained, which is the one in which the signal originated by the product of the reaction differs from the background. For replicates of each sample, the average Ct was calculated. The ΔCt was calculated using the formula Δ^{Ct}= Ct_{Exon6} - Ct_{TFRC}. Normalization was carried out by calculating the ΔΔCT (ΔΔCt = ΔCtₛₐₘₚₗₑ - ΔCt_{reference}). The RQ of each sample was calculated following the formula: RQ = 2^{-ΔΔCt}.

### Cytometry

Once the mouse mammary glands were removed, they were incubated in a 50 mL tube containing 9 mL of medium (Gibco DMEM/F-12, #11554546, Fisher Scientific), 500 µL of collagenase and 500 µL of hyaluronidase, at a temperature of 37 °C for 16 hours. After incubation, the aggregated tissue debris was disrupted by pipetting for approximately 30 seconds and the sample was centrifuged for 5 minutes at 350 g at room temperature. Centrifugation was performed, in all examples, using the Eppendorf 5804R refrigerated centrifuge including an A-44 rotor with a radius of 15 cm (width (metric) exterior 634 mm; depth (metric) 550 mm, height (metric) exterior 342 mm). The supernatant was discarded and the pellet formed was resuspended in 2 mL of ACK buffer (Gibco ACK Lysing Buffer, #A10492-01, Fisher Scientific) to lyse the erythrocytes present in the sample. The disaggregated sample was incubated in ACK buffer for 5 minutes at room temperature and subsequently centrifuged at 350 g for 5 minutes. After centrifugation, the supernatant was discarded and 1 mL of trypsin was added at 37 °C. The pellet was resuspended by pipetting for 2 minutes. Next, 10 mL of saline (Gibco HBSS, #14025-092, Fisher Scientific) supplemented with 10 mM HEPES buffer and fetal bovine serum was added and centrifuged for 5 minutes at 350 g at room temperature. Then, the supernatant was discarded and 1 mL of the buffered saline solution (hereinafter HF) and 100 µL of warm DNase were added, carefully pipetted for 1 minute and 5 mL of cold HF was added, to stop action of the DNase. The mixture was filtered through a 40 µm filter and washed with 5 mL of cold HF. It was centrifuged for 5 minutes at 350 g, at a temperature of 4 °C and the supernatant was removed after centrifugation. Subsequently, the mixture was resuspended in 5 mL of cold HF and the mixture was distributed in cytometry tubes with a 35 µm filter. 4/5 of the sample was allocated for immunolabeling and the remaining 1/5 was allocated as an unlabelled control. It was centrifuged for 5 minutes at 1200 rpm (= 20 s⁻¹) at a temperature of 4 °C and the supernatant was decanted after centrifugation. Subsequently, 52 µL of blocking solution, consisting of 50 µL of HF and 2 µL of anti-CD16/CD32 antibody, were added, gently vortexed and incubated for 10 minutes at 4 °C. Next, 50 µL of immuno-labelling solution made up of the following antibodies: CD45 FITC, CD140 FITC, CD31 FITC, Ter119 FITC, CD49fAPC, CD29 APC-Cy7, CD61 BV421, Sca1 PerCP-Cy5, EpCAM PE- Cy7, e506, was added to the tube intended for staining and 50 µL of HF was added to the control tube. After incubating for 30 minutes and performing a wash, the samples were acquired with the LSR Fortessa X-20 Cell Analyzer cytometer (BD Biosciences). The analyses were carried out with the Flow Jo software.

### Separation of RFP+ breast cells by FACS sorting

The same samples collected to analyse the epithelial subpopulations of the mammary gland were used to separate RFP+ and RFP- cells. Approximately 100,000 to 500,000 epithelial cells expressing RFP were separated with the Fluorescence-Activated Cell Sorting (FACS) Aria III Cytometer after excluding debris, doublets, dead cells, and stromal cells. These cells were collected in PBS, centrifuged for 15 minutes at 4 °C and 1800 rpm (= 30 s⁻¹), and stored for later RNA extraction.

### RNA extraction from RFP+ cells

RNA extraction from RFP+ cells was performed with the commercial RNeasy mini kit (#74104, Qiagen). The frozen cells were tempered and added to a falcon with solution A (80 mL of FBS, 4000 U of heparin, 100 mg of DNAse, 300 mL of DMEM) previously tempered. After centrifugation at 1200 rpm (= 20 s⁻¹) for 5 minutes, the supernatant was removed and the RLT buffer was added together with *beta*-mercaptoethanol. These cells were then transferred to QiAshredder columns to homogenize the lysate for 2 minutes at maximum speed. One volume of 70% ethanol was added to the lysate that passed the column, and after mixing with the pipette, 700 µL were transferred to an RNeasy column. After centrifuging for 15 seconds at 12,000 rpm (= 200 s⁻¹), the liquid that passes into the collection tube was discarded. Next, 700 µL of RWT buffer was added and the column was centrifuged at 12,000 rpm for 15 seconds. Next, two washes were performed with 500 µL buffer RPE and centrifugation at 12,000 rpm (= 200 s⁻¹) for 15 seconds for the first wash and for 2 minutes for the second. Finally, 30 µL of RNAse-free water was added to the centre of the column to dilute the RNA and after centrifugation, the tube was left on ice to prevent degradation.

### RNA-seq

The RNA-seq processing and analysis was carried out with the company Novogene Europe (UK). Briefly, before making the libraries, the RNA samples were quantified by nanodrop to measure the quality of the RNA (OD260/OD280), they were run on agarose gel to evaluate degradation and potential contamination, and it was measured on an Agilent 2100 chip to assess the integrity of the RNA. Subsequently, the libraries were built, with an enrichment of the mRNA, synthesis of doublestranded cDNA, addition of adapters. For the evaluation of the quality of the libraries, the concentration of the libraries was measured by fluorimetry using the Qubit 2.0 fluorometer (Life Technologies), the size of the fragments was evaluated using Agilent 2100 and the quantification of the fragments using QPCR and sequencing using a system Illumina PE150 and a read depth of 15G.

For the RNA-Seq analysis, the first thing was filtered and poor-quality reads were discarded (with adapter contamination, or with 10% or more of indeterminate nucleotides or with abundance of poor quality nucleotides, of 50% or more). The filtered reads were aligned with the reference genome using specific algorithms (HISAT2, version V2.0.5). The mapped regions were enriched in exonic zones by more than 90%, as would be expected. For the quantification of the expression, FPKM units (fragments per kilobase per million base pairs sequenced) were used, which allows comparing the expression levels between genes and experiments, considering not only the fragment count, but also the length of the gene. and the depth of sequencing (all factors that influence expression levels). Quantification was carried out with the HTSeq algorithm version v0.6.1.

Significantly expressed genes were those that exceeded an FPKM threshold of 0.3. Gene co-expression between samples was represented by Venn diagrams. Differential expression analysis for samples with biological replica was DESeq2 version v.10.0 and in cases without replica, DEGSeq version 1.34.1. To identify groups of genes that could be involved in the same function, FPKM-cluster analyses were carried out, with the same purpose clusters were made with log2ratio measurements using H-clusters, K means and SOM. Enrichment studies were carried out to identify biological functions and pathways significantly associated with differentially expressed gene pools. To do this, Gene Ontology (GO) analysis was used (using GOSeq, topGO, semmscan algorithm, version v.12) and KEGG (Kyoto Encyclopedia of Genes and Genomes) using KOBAS version v3.0 software.

### Statistical analysis

The comparison of temporal variables was performed with Kaplan-Meier curves and the level of significance with the Log-Rank test. In the comparison between proportions, the Chi-square test or

Fisher's exact test was used. To study the differences in the average value of a specific phenotype between two samples or groups, the non-parametric Mann Whitney U test was used, given that the samples did not follow a normal distribution (normality was evaluated by the Kolmogorov-Smirnov test). When more than two groups were compared, the Kruskal-Wallis test was used for non-normal populations. After performing the global comparison, a *post-hoc* test (usually the Dunn test) was used, which identified the groups among which there were statistically significant differences. The correlation between continuous variables was studied using the Pearson test in the event that the variables followed a normal distribution. Otherwise, the Spearman test was used. The level of significance was established at *P* ≤ 0.05. The statistical programs used to perform the described tests were GraphPad Prism5 and JMP 12.

### Example 1. Pregnancy increases resistance to breast cancer in the Brca1/P53 deficient mouse model

Early and repeated pregnancy is one of the most resistant factors inducing breast cancer. Therefore, we first evaluated the extent to which pregnancy induces resistance to breast cancer in the mouse model deficient in *Brca1*/*P53* in the breast. The female mice endured two rounds of pregnancy and lactation, and latency and tumour incidence were compared in nulliparous and multiparous animals and, within the latter, those that provided and did not provide lactation.

It was observed that mice that were pregnant developed tumours with a longer latency than nulliparous mice (P < 0.0001) **(****Figure 1A****).** The delay in the appearance of the tumour with respect to the nulliparous mice was present both in the mice that did not lactate (P = 0.0009), and in those that did lactate (P = 0.0002). No differences in tumour latency were observed between mice that lactated and those that did not.

Regarding tumour incidence, defined as the percentage of animals that developed mammary tumours throughout their lives, a statistically significant decrease was observed in multiparous compared to nulliparous mice (P = 0.011) **(****Figure 1B****).** Thus, while the tumour incidence in nulliparous mice was 88.7% (31 mice out of 35 developed mammary tumours), in multiparous mice without lactation it was 63.6% (21 mice out of 33) and in the lactation group it was 59.4% (19 mice out of 32).

Therefore, the protective effect of pregnancy is preserved in the study model deficient in *Brca1*/*P53;* and a priori it is a useful model to evaluate drugs that can enhance this protective effect of pregnancy.

### Example 2. Cabergoline potentiates the protective effect of pregnancy in the Brca1/P53 deficient mouse model

The effect of cabergoline on breast cancer susceptibility was evaluated in Brca1/P53-deficient mice after pregnancy. It was found that cabergoline administered in a single dose, after the second pregnancy and weaning, was associated with greater latency, with a statistically significant difference between the groups (P < 0.0001) **(****Figure 2A****).** Specifically, treatment with cabergoline increased the protective effect observed in the pregnancy and lactation group (P = 0.019), the non-lactation pregnancy group (P < 0.0001) and, with much greater significance, with respect to the nulliparous group (P < 0.0001).

A protective effect of cabergoline treatment was also observed with respect to the incidence of breast cancer (P < 0.0001). So that in the group treated with cabergoline the incidence was 27.78% (10 mice out of 37 developed tumour) compared to 59.4% (19 mice out of 31 developed tumour) in the multiparous and lactation group, and 88.7% (31 mice out of 35 developed tumour) in the nulliparous group **(****Figure 2B****).** The tumour incidence of the group treated with cabergoline was significantly lower than that of the lactating multiparous mice (P = 0.0062).

In conclusion, cabergoline potentiates the protective effect of pregnancy in the *Brca1*/*P53* deficient model in terms of latency and tumour incidence.

### Example 3. Cabergoline potentiates post-lactational involution in female mice deficient in Brca1/P53

During pregnancy there is a massive proliferation of the epithelial cells of the mammary glands. With post-pregnancy/post-lactational involution, massive apoptosis of the epithelial cells occurs, which fall into the lumen of the ducts and is maximal in mice on day 3 post-weaning. We evaluated the extent to which cabergoline potentiated post-lactational involution in Brca1/P53-deficient mice.

Therefore, we evaluated apoptosis on days 1, 3, and 7 after weaning in multiparous mice treated and untreated with cabergoline. The study confirmed a significant increase in apoptosis in mice treated with cabergoline at 3 days of involution (P < 0.0001) **(****Figures 3A** to **3F****),** demonstrating that cabergoline induces a more potent post-lactational involution process.

### Example 4. Cabergoline accelerates histological changes characteristic of post-lactational involution in female mice deficient in Brca1/P53

During post-pregnancy/post-lactational involution, increased epithelial cell apoptosis causes a progressive decrease in the number of ducts and, simultaneously, interductal adipose tissue reappears. This translates into a progressive increase in the area of adipose tissue.

It was observed that at 3 days of involution in the group treated with cabergoline, the interductal adipose component is already more clearly observed, while it was barely present in the absence of treatment **(****Figure 4A****).** The quantification of the adipose tissue area showed that the group treated with cabergoline exhibited a statistically significant increase in adipose tissue at 3 and 7 days after weaning (P < 0.001, in both cases) **(****Figures 4B** to **4G****).** This result demonstrates that treatment with cabergoline accelerates post-lactational involution.

In conclusion, the increase in apoptosis and interductal adipocyte area demonstrates that cabergoline potentiates and accelerates post-lactational involution in *Brca1*/*P53*-deficient mice.

### Example 5. In the long term, cabergoline produces a significant reduction in the epithelial component of the mammary gland

Since cabergoline induced greater and accelerated post-lactational involution, it was evaluated whether this had an impact on the amount of epithelial tissue in the breast in the long term, once involution was complete. Therefore, the ductal epithelial component was quantified at 30 and 60 days after weaning using an algorithm.

At 30 days post-lactational involution, it was observed that the group treated with cabergoline had a drastic decrease in ductal area. Another series of facts is striking: (i) a lot of residual ductal epithelium still remains in the multiparous and lactating group; (ii) in nulliparous mice, an increase in the epithelium was observed with age from 3 to 4 months **(****Figure 5A****).**

Since a lot of residual epithelium still remains, it was again evaluated at 60 days post-lactational involution. Here again, the group treated with cabergoline maintained a great reduction of the epithelial component **(****Figures 5B** and **5C****).** Comparing the epithelial components at 30 and 60 days, it was observed that (i) cabergoline maintains the decrease in the ductal component; (ii) the lactating multiparous group has significantly decreased ductal area; (iii) the group of multiparous mice with lactation paradoxically has increased the ductal area, probably because it is more delayed in involution; and (iv) the increase in ductal area in nulliparous mice is observed **(****Figure 5C****).**

In conclusion, cabergoline induces a decrease in the parietal ductal area and, with it, in the epithelial component of the breast. This is important, given that this breast tumour derives from the epithelial component of the gland and here it is significantly reduced. Thus, the decrease in the number of cellular targets with the capacity to become malignant could explain the protective effect of cabergoline.

### Example 6. Cabergoline accelerates the appearance of a hypoproliferative state in the mammary epithelium

Since a smaller ductal component was observed in the mammary glands of mice treated with cabergoline at 30 and 60 days post-weaning **(****Figure 5****),** epithelial proliferation was then assessed at those time points by detection of Ki67 labeling (immunohistochemistry) and subsequent quantification with an algorithm **(****Figure 6****).** Thus, it was observed that at 30 days post-weaning, cabergoline induced a profound hypoproliferative state **(****Figure 6A****)** that could contribute to the decrease in the epithelial component of the breast observed at 30 and 60 days post-weaning **(****Figure 5****).**

At 60 days post-weaning, a significant decrease in proliferation was observed in all groups that were subjected to pregnancy, with or without lactation, without significant differences between groups. While in *Brca1*/*P53* deficient mice that did not have pregnancy, a progressive increase in cell proliferation was observed at the equivalent age of 5 months **(****Figure 6B****).**

Here we observe that in mice treated with cabergoline the hypoproliferative state is induced earlier than in the other groups with pregnancy. This hypoproliferative state may be justified by the downregulation of multiple proliferation pathways.

### Example 7. The mammary glands of mice treated with cabergoline show greater basal apoptosis in the epithelial component at 30 days post-weaning

Since a lower ductal component was observed in the mammary glands of mice treated with cabergoline at 60 days, the basal apoptosis of the epithelium was evaluated before reaching that moment, at 30 days post-weaning **(****Figure 7****).** Thus, a greater basal apoptosis of the epithelium was observed, which may also have contributed to the decrease in the epithelial component of the breast, observed at 60 days post-weaning **(****Figure 5****).**

In conclusion, the mammary glands of mice treated with cabergoline present a smaller epithelial component at 30 and 60 days after treatment **(****Figure 5****),** which may have been achieved: (i) by a more powerful and accelerated post-lactational involution that eliminated more epithelial cells **(****Figures 3** and **4****);** (ii) to a lower state of basal proliferation that is evident at 30 days after treatment **(****Figure 6****);** (iii) increased basal apoptosis that is also present at 30 days post-lactational involution **(****Figure 7****).** An expected consequence of the lower epithelial component of the mammary glands of mice treated with cabergoline is that they also have a lower proportion of recombinant (potentially tumour-initiating) cells. In this regard, it may be recalled that the model describes 20-30% of recombinant cells (which lose *Brca1*/*P53)* and that they are the ones from which the *Brca1*-deficient tumour in patients derives.

The following examples are aimed at evaluating to what extent there are fewer recombinant cells after treatment with cabergoline.

### Example 8. The proportion of recombinant cells is maintained at 30 and 60 days post-weaning, which indirectly indicates a reduction in the absolute number of recombinant cells in the groups with reduced epithelial component

As indicated above, in this model *P53* and *Brca1* are not lost in 100% of breast cells, but rather in 20-30% of them. It should not be forgotten that breast cancer derives from these recombinant cells. Therefore, it was evaluated if there were differences in the proportion of recombinant cells between groups. The qPCR technique was used to evaluate the proportion of recombinant cells in the breast of the different groups at 30 and 60 days post-weaning/pregnancy. However, due to logistical issues of difficulty in designing efficient primers to detect the recombinant form, the non-recombinant allele or "wild type" (WT) was quantified using TaqMan probes. Since 100% of the alleles are made up of the sum of the WT allele plus recombination, changes in the percentage of the WT allele, would indirectly also indicate changes in the recombined allele.

The quantification of the non-recombinant allele (wild type or WT) by means of qPCR, at 30 and 60 days of post-lactation involution (cross-sectional cohorts), did not show statistically significant differences between groups **(****Figures 8A** and **8C****).**

The absence of differences in the amount of normal allele in non-tumour tissues also indicates that there are no significant differences in the proportion of normal cells between groups and, therefore, neither in the proportion of recombinant cells. This does not mean that there is no decrease in the absolute number of recombinant cells. Just the opposite, as we have seen that at 30 and 60 days there is a significant reduction in the ductal (epithelial) component in the group treated with cabergoline **(****Figure 5****),** if there are no changes in the proportion, this means that there will be a decreased proportion of recombinant cells in absolute terms.

### Example 9. The quantification of the recombinant cells with fluorescence at 60 days of involution confirms the lack of statistically significant differences in their proportion between groups

To confirm the absence of significant differences between groups in the proportion of recombinant cells, a direct method of detection of these was employed. Thus, the proportion of recombinant cells was directly evaluated by detecting the fluorescence emitted by them. To do this, mice deficient in *Brca1*/*P53,* carriers of Cre-recombinase under the cytokeratin 14 promoter, were crossed with mice carrying red fluorescent protein (RFP) under the promoter of ROSA26. However, in the model, the RFP cDNA is preceded by a stop codon, flanked by *LoxP* sites, which prevents transcription of the fluorescent protein. After several crosses, mice deficient in *Brca*/*P53* in the breast were generated, in whose recombinant cells the Cre recombinase not only inactivates *Brca1* and *P53,* but also eliminates the stop codon and the recombinant cells emit red fluorescence **(****Figure 9A****).** For its quantification we carried out flow cytometry **(****Figure 9B****).**

In the quantification of the Lin-, RFP+ recombinant cells, no significant differences were observed in their proportion between groups at 60 of involution **(****Figure 9C****).** In order not to identify epithelial cells only by exclusion (i.e. those which are not stromal cells: Lin+, RFP+), the percentage of EpCAM+ RFP+ cells (EpCAM is a pan-epithelial marker) within epithelial cells (Lin-) was also estimated and no significant differences were observed **(****Figure 9D****).** This coincides with the allelic quantification previously carried out using qPCR **(****Figure 8****).**

The data observed at 60 days of involution by qPCR and FACS show that there are no significant differences between groups in the percentage of recombinant cells. As we discussed earlier, this does not mean that there is no decrease in the absolute number of recombinant cells. Just the opposite, as we have seen that at 30 and 60 days there is a significant reduction in the ductal epithelial component **(****Figure 5****).** If the ductal area at 60 days is corrected by the percentage that would correspond to recombinant cells (EpCAM+ RFP+), the area due to epithelial recombinant cells can be roughly estimated and allow its reduction to be estimated in absolute terms **(****Figure 9E****).**

### Example 10. Pregnancy produces a decrease in the proportion of the luminal component, within the recombinant epithelial cells, without significant differences with the group treated with cabergoline

Given that the tumour is derived from the recombinant cells which lose *Brca1* and *P53,* and which are marked with red fluorescence in this model, it was evaluated whether there were quantitative changes in subpopulations within the recombinant (RFP+) cells between the study groups at 60 days after weaning and its equivalent age in nulliparous mice. In addition, to rule out that the cells that lose *Brca1* and *P53* have a different behaviour than the normal ones, control or "wild type" (WT) mice for *P53* and *Brca1* were introduced into the experiment, but that express RFP in the same cells cytokeratin 14 positive (WT-RFP+).

In the breast there are two large subgroups of epithelial cells, those that are towards the lumen of the tubules or luminal cells, and those that are outside of these, next to the basal membrane, are the basal cells and are myoepithelial in nature **(****Figure 10A**)**.** These two subpopulations can be distinguished (i) by the level of expression of the pan-epithelial marker EpCAM, which is higher in luminal cells, and (ii) of the CD49f marker, which is more expressed in basal cells. Thus, luminal cells can be defined as EpCAM high (hi)/CD49f low (lo) and basal cells as EpCAM lo/CD49f hi **(****Figures 10B** and **10C****).**

Thus, we observed that in multiparous mice which have weaned their offspring, both with and without treatment with cabergoline, there was a reduction in the luminal component and a proportional increase in the basal component, within the recombinant cells (RFP+) with respect to the group of nulliparous mice. Therefore, although no differences were seen in the percentage of RFP+ epithelial cells, changes were observed in their proportion of luminal (decrease) and basal (increase) **(****Figures 10D** and **10E****).** The behavior of WT-RFP+ and *Brca1*/*P53* deficient (KO-RFP+) mice was similar, indicating that the observed effect is due to pregnancy and not to the sum of its effects and the loss of *Brca1* and *P53* **(****Figures 10D** and **10E****).**

No significant differences were observed between multiparous mice treated with cabergoline and untreated multiparous mice. But given that the total ductal component is significantly lower after treatment with cabergoline at 60 days **(****Figure 5****),** this should have repercussions, in absolute terms, not only in a lower recombinant epithelial component (RFP+) **(****Figure 9E****),** but also, within it, in the luminal.

The reduction of the luminal component within recombinant cells is of importance, as *Brca1*-deficient breast cancer has been shown to originate from luminal precursors. Therefore, very importantly, the decrease in the luminal component within the recombinant cells suggests that pregnancy decreases the number of potential cellular targets that are the origin of the tumour, and, in absolute terms, this decrease would be even greater after treatment with cabergoline.

### Example 11. Pregnancy produces a decrease in the luminal ductal component within the recombinant epithelial cells, without significant differences with the group treated with cabergoline

Within the luminal cells there are two major subpopulations, the ductal (including mature ductal and progenitor cells) and alveolar (including early progenitor, late progenitor, and mature alveolar cells) **(****Figure 11A****).** The former form ducts that conduct the milk produced by the alveolar cells during lactation, mainly the mature alveolar cells. During post-lactational involution, a large amount of the luminal component that was generated during pregnancy and lactation is lost, both the ductal cells (arborizations) and the alveolar cells, but especially the latter. After said involution, the arborizations are reduced, leaving fewer ducts; and mature milk-producing alveolar cells are also eliminated, leaving only early alveolar precursors.

Thus, it was evaluated whether the decrease in the luminal component after pregnancy equally affected ductal (Sca1+) and alveolar (Sca1-) cells or whether there were differences between the groups studied. Therefore, any differences in the proportion of Sca1 cells within the subpopulation of recombinant (RFP+) total luminal cells (EpCAM hi/CD49f lo) were determined **(****Figure 11B**). Interestingly, pregnancy produced a reduction in the proportion of luminal ductal cells within the total number of recombinant luminal cells and, with it, a relative increase in alveolar precursors **(****Figures 11C** and **11D****),** with no significant differences between the multiparous groups, namely multiparous mice treated with cabergoline and untreated multiparous mice. The similar effect in WT-RFP+ and *Brca1*/*P53* deficient (KO-RFP+) mice, again demonstrates that this is an effect of pregnancy **(****Figures 11C** and **11D****).**

In addition, it cannot be forgotten that the changes observed in ductal and alveolar cells with respect to the total number of luminal cells are relative, since both populations add up to the total number of luminal cells. When the changes observed with respect to total recombinant epithelial cells (RFP+) are considered, the decrease in luminal ductal cells is maintained **(****Figure 11E**), but the increase in luminal alveolar cells is no longer observed, there are no significant differences between groups **(****Figure 11F**).

But again, given the decrease in ductal area, in absolute terms, in the group treated with cabergoline at 60 days **(****Figure 5****),** the absence of differences between proportions between multiparous mice without treatment and with cabergoline treatment should have repercussions, in absolute terms, not only in a minor recombinant epithelial component (RFP+) **(****Figure 9E****),** but also in the luminal, therewithin. Since luminal ductal cells have been related to being the targets of *Brca1*-defective tumors, their decrease would help explain the combined protective effect of pregnancy and of cabergoline.

### Example 12. Cabergoline induces a significant relative increase in luminal ductal progenitors within RFP+ recombinant cells, but without differences with respect to total recombinant cells.

Differences within the ductal population, specifically, between the proportions of progenitor (CD61+) and mature (CD61-) luminal ductal cells **(****Figure 12A****)** were explored. No differences were observed between nulliparous and lactating/breastfeeding multiparous mice (i.e. which have weaned their offspring). A relative increase in precursors and, in parallel, a relative decrease in mature ductal cells, with respect to total recombinant ductal cells, was observed in the group treated with cabergoline compared to the other two groups. Effect that was only seen in the KO-RFP+ group, not in the WT-RFP+ group **(****Figures 12B** and **12C****).**

Since the observed changes are relative insofar as both subpopulations of ductal cells (progenitors and mature) form 100% of them, we then evaluated the proportion of the ductal cell subpopulations with respect to the total of recombinant epithelial cells. Thus, we observed that both luminal and mature ductal precursors decreased in multiparous mice, namely both multiparous mice treated with cabergoline and untreated multiparous mice, compared to nulliparous mice. Furthermore, that there are no statistically significant differences between the aforementioned two groups of multiparous mice, **(****Figures 12D** and **12E****).**

As with the other populations, since no significant differences were observed in the proportions of ductal cells between multiparous mice treated with cabergoline and untreated multiparous mice, given that the total ductal component is significantly lower after treatment with cabergoline at 60 days **(****Figure 5****),** this should have repercussions, in a decrease in absolute terms in the group treated with cabergoline, not only in a lower recombinant epithelial component (RFP+) **(****Figure 9E****),** but also, within this, in the luminal components, in the ductal and in the two ductal subpopulations.

### Example 13. Pregnancy and cabergoline affect the proportion of recombinant cells over time

The studies carried out so far are a "snapshot" at 30 or 60 days of post-lactational involution. It was thought possible to assess changes in the proportion of recombinant cells over time. For this reason, the proportion of recombined cells in the mice that developed tumours at different times (herein referred to as the "longitudinal cohort") were evaluated. The longitudinal cohort was made up of mice of different ages, depending on when they had to be sacrificed due to the appearance of the breast tumour or for other reasons.

Again, for logistical reasons, the degree of chimerism was detected by evaluating the WT allele using qPCR and TaqMan probes. Differences were observed in the proportion of normal cells and, therefore, of the recombinant cells over time **(****Figure 13****).** The group that presented fewer normal cells and a decrease over time (and, therefore, the one that had more recombinant cells) with a statistically significant negative correlation, was that of the nulliparous mice **(****Figure 13A****).** A similar decrease in WT cells (progressive increase in the number of recombinant cells) was then observed in the non-lactating multiparous group **(****Figure 13B****).** Interestingly, in the lactating multiparous group, no significant changes were observed in the proportion of WT cells (and thus also in recombinant cells) over time **(****Figure 13C****).** Finally, in the group of multiparous mice treated with cabergoline, an increase in WT cells was observed over time, which would indicate a parallel decrease in recombinant cells **(****Figure 13D****).** This last result is surprising.

By way of a conclusion to the aforementioned Examples, cabergoline produces an earlier and more powerful post-lactational involution, and advances the antiproliferative effect of pregnancy and increases the basal apoptosis of the epithelium. All this produces a significant reduction in the ductal surface that is greater than that seen after pregnancy/lactation **(****Figure 5****).** It has not been observed that cabergoline produces specific changes in epithelial subpopulations with respect to the group having undergone pregnancy and lactation. The same subpopulations are affected as with pregnancy, fundamentally a reduction in the luminal ductal population **(****Figures 11** and **12****)** which is where the cellular targets originating from the tumour are found. However, the greater total epithelial reduction that occurs with treatment with cabergoline means that it is in this group where, in absolute terms, the greatest reduction in tumour-initiating targets occurs.

### Example 14. Cabergoline modifies transcription: RNA-sequencing study

Pregnancy produces epigenetic changes in the epithelium of the mammary gland that affect its behaviour and probably also influence pregnancy-induced tumour susceptibility/resistance. Here this has been observed, for example, in the changes in proliferation and basal apoptosis, and in the epithelial subpopulations induced by it. To evaluate changes in expression induced by cabergoline, a preliminary RNA-sequencing study was carried out in RFP+ cells from the different study groups isolated by flow cytometry (FACS sorting) 60 days post-lactation **(****Figure 14****).**

Thus, pre-amplification was performed, and RNA-sequencing was carried out. A series of common and condition-specific genes were found **(****Figures 14A** to **14C****).** The differential genes allowed formation of an unsupervised cluster. In this study, the KO-nulliparous mice group was isolated from the rest, and then the multiparous WT mice group treated with cabergoline also formed another group, but closer to the KO group who were pregnant. All the KOs that went through the pregnancy clustered together. Of those treated with cabergoline, two were left together and one next to the untreated one **(****Figure 14D****).** Differential genes were grouped by function using Gene Ontology. The expression changes indicate that cabergoline contributes to epigenetic changes in the breast.

## Claims

1. Cabergoline or a pharmaceutically acceptable salt or solvate thereof, for use in prevention or delay of post-pregnancy breast cancer in a female mammal.

2. Cabergoline or a pharmaceutically acceptable salt or solvate thereof, for use according to claim 1, wherein said female mammal has weaned their offspring.

3. Cabergoline or a pharmaceutically acceptable salt or solvate thereof, for use according to any one of claims 1 and 2, wherein said cabergoline or said salt or solvate is administered to said female mammal during and/or after post-lactational involution.

4. Cabergoline or a pharmaceutically acceptable salt or solvate thereof, for use according to any one of claims 1 to 3, wherein said female mammal is a woman.

5. Cabergoline or a pharmaceutically acceptable salt or solvate thereof, for use according to claim 4, wherein said woman is ≥ 30 years old when having first given birth.

6. Cabergoline or a pharmaceutically acceptable salt or solvate thereof, for use according to claim 5, wherein said woman is ≥ 35 years old when having first given birth.

7. Cabergoline or a pharmaceutically acceptable salt or solvate thereof, for use according to any one of claims 4 to 6, wherein said woman was pregnant for more than 34 weeks.

8. Cabergoline or a pharmaceutically acceptable salt or solvate thereof, for use according to any one of claims 1 to 7, wherein said female mammal has a mutation in the *Brca1* gene and/or the *P53* gene.

9. Cabergoline or a pharmaceutically acceptable salt or solvate thereof, for use according to any one of claims 1 to 8, wherein said post-pregnancy breast cancer is a ductal epithelial breast cancer.
